# EUROPEAN PATENT SPECIFICATION

(11) **EP 1 875 854 B1**
(45) Date of publication and mention of the grant of the patent: **02.11.2011**
(21) Application number: 07012944.0
(22) Date of filing: 02.07.2007
(51) Int. Cl.: A61B 1/018, A61B 18/14, A61B 17/28, A61B 17/32

(54) **Treatment instrument for endoscope**
Endoskop-Behandlungsinstrument
Instrument de traitement pour endoscope

(30) Priority: 05.07.2006 JP 2006185637
(43) Date of publication of application: 09.01.2008
(73) Proprietor: OLYMPUS MEDICAL SYSTEMS CORP., Tokyo 151-0072 (JP)
(72) Inventor: Yutaka Yanuma c/o Olympus Medical Systems corp, Tokyo (JP)
(74) Representative: von Hellfeld, Axel

(56) References cited:
- WO-A-02/11621
- WO-A-03/096880
- JP-A- 3 139 340
- US-A- 5 396 900

## Description

### BACKGROUND OF THE INVENTION

### Field of the Invention

The present invention relates to a treatment instrument for endoscope which is used by passing through the endoscope.

### Description of Related Art

In recent years, cases for endoscopically giving medical treatment to gastrointestinal system and pancreaticobiliary system have been increasing. For example, inserting a catheter from the duodenal papilla and performing fluoroscopy relative to the bile duct or pancreatic duct for diagnosis, or treatment for retrieving gallstone by balloon or basket constitute endoscopical medical treatment for pancreaticobiliary system.

However, in order to retrieve the gallstone, since the openings at the exits of the duodenal papilla and the bile duct are narrow, a high-frequency knife called the papillotome needs to be used to cut out the exits of the duodenal papilla and the bile duct to widen the opening (for example, see Japanese Unexamined Patent Application, First Publication No. 2001-70316). The high-frequency knife has a constitution in which a conductive wire is inserted through a flexible sheath of fine diameter capable of being inserted in the exits of the duodenal papilla and the bile duct, and the wire is exposed to the outer wall face at the distal end portion of the flexible sheath. When used, the flexible sheath is bent into a curve with the distal end portion of the flexible sheath being inserted in the bile duct from the duodenal papilla. As a result, since the distal end portion of the high-frequency knife becomes bow shaped by the wire as a chord, when high frequency electric current is applied to the wire, the sphincter muscle of the duodenal papilla is cut out. As a result, the exits of the duodenal papilla and bile duct become wider.

Incidentally, to cut out and widen the exits of the duodenal papilla and the bile duct, the duodenal papilla should be cut out along the direction in which the bile duct extends. Also, the cut-out should be performed in an appropriate direction and over the appropriate cut-out length to ensure that bleeding during cut-out is inhibited. The appropriate cut-out direction and cut-out length are generally decided from the anatomical bile duct direction and from the positions of blood vessels in its surroundings. With regard to the cut-out direction, observing from the front of the duodenal papilla, if the mouth side of the duodenum is in the 12 o'clock direction and the anus side is in the 6 o'clock direction, then the cut-out direction locates in the 11 o'clock to 12 o'clock direction when the opening of the duodenal papilla is taken as the center of the direction. And the cut-out length is, at the maximum, up to the upper border of the protuberance on the mouth side of the duodenal papilla. If cut out in a direction other than 11 to 12 o'clock direction, or if cut out to a length longer than the upper border of the protuberance on the mouth side of the duodenal papilla, the probability of bleeding will increase.

Accordingly, the cut-out length and cut-out direction by the high-frequency knife should preferably be adjustable according to the need of the operator. However, in case of the conventional high-frequency knife, the direction and length of cut-out had to be adjusted by finely operating the endoscope during the input of high frequency electric current, that is, during the cut-out, and thus, experienced skill was required for using the conventional knife.

Also, for cutting out the exits of the papilla and the bile duct with the conventional papillotome, insertion of the flexible sheath mentioned above in the bile duct was indispensable for adjusting the cut-out length and the cut-out direction.

However, sometimes it was not possible to insert the treatment instrument in the bile duct, depending on how the opening in the bile duct or the opening of the papilla of the pancreatic duct was formed.

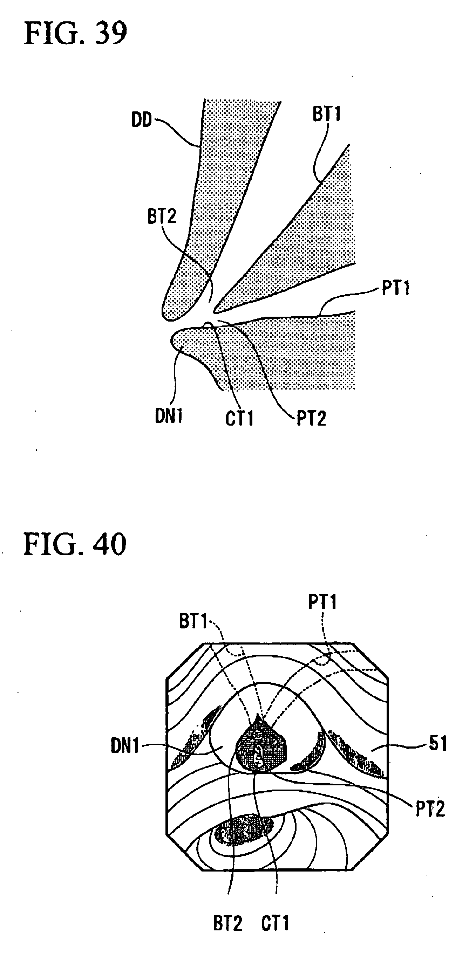

For instance, as shown in FIG. 39, the internal portion of the papilla is constituted by a tube called a common channel CT1 which extends from the opening of the papilla DN1 of the duodenum to the back thereof. The openings BT2, PT2 of the bile duct BT1 and pancreatic duct PT1 respectively, are often located at the back of the common channel CT1. While the entrance PT2 of the pancreatic duct PT1 extends in a substantially perpendicular direction relative to the wall of the duodenum, the bile duct BT1 is above the pancreatic duct PT1 (in the direction of the mouth side of the duodenum), that is, it extends at an angle in the opposite direction relative to the direction of insertion of the endoscope. Thus, the treatment instrument protruding from the endoscope sometimes enters the pancreatic duct PT1, direction of which is easier for the treatment instrument to be adjusted, and cannot enter the bile duct BT1, direction of which is difficult for the treatment instrument to be adjusted.

When the papillotome cannot be inserted in the bile duct, as noted in the Japanese Unexamined Patent Application, First Publication No. H04-329944, such procedure is performed that a needle-shaped high-frequency knife is used and the entrance of the papilla is cut out (pre-cut) such that the entrance BT2 of the bile duct BT1 and the entrance PT2 of the pancreatic duct PT1 are separated as shown in FIG. 40, so that the tool can be inserted in the bile duct BT1. However, the cut-out by the needle-shaped high-frequency knife requires adjustment of the position of the distal end of the needle by endoscopic operation only. Thus, compared to the cut-out by papillotome performed with the distal end of the sheath inserted in the bile duct BT1, it is a procedure more difficult in adjusting the cut-out direction, the cut-out length, and the cut-out depth. Therefore there has been a problem that only a few operators with very experienced skills in endoscope operations could make such procedure.

WO 02/11621 A1 describes a treatment instrument for an endoscope featuring two forceps pieces at the distal end of the insertion portion.

The main object of present invention is to facilitate and ensure cut-out of openings formed in biological tissues considering the circumstances described above.

### SUMMARY OF THE INVENTION

A treatment instrument for an endoscope achieving said object in accordance with the present invention is described in claim 1.

Preferred embodiments of the treatment instrument are described in the dependent claims.

In the first aspect of the treatment instrument for endoscope, the distal end insertion portion is inserted in the entrance of the lumen of the body with the first forceps piece opened. The cut-out amount is adjusted by the insertion amount of the distal end insertion portion, and the cut-out position is adjusted by the direction of the first forceps piece. The biological tissue is held between the first forceps piece and the distal end treatment portion, high frequency electric current is applied, and the biological tissue is cut out. If necessary, the first forceps piece and the distal end insertion portion are inserted in the lumen from the cut out entrance. A contrast agent is injected, or a guide wire is inserted through the distal end insertion portion. The guide wire may be inserted in the lumen beforehand, and the distal end insertion portion may be inserted in the entrance of the lumen along the guide wire.

The second aspect of the invention is the treatment instrument for endoscope according to the first aspect of the invention, in which the insertion portion has two or more lumens; the first lumen is communicated with the distal end insertion portion and opens on the proximal end side of the insertion portion; the second lumen is inserted by an operation wire which freely advances and retracts thereinside for operating the first forceps piece.

In the second aspect of the treatment instrument for endoscope, the first lumen, which is independent of the second lumen through which the operation wire passes, is inserted by the guide wire and the contrast agent, the operation wire and the guide wire do not interfere with each other.

The third aspect of the invention is the treatment instrument for endoscope according to the second aspect of the invention, in which the insertion portion has a third lumen; the third lumen opens on the proximal side of the insertion portion, and is communicated with the first lumen at the distal end side of the insertion portion.

In the third aspect of the treatment instrument for endoscope, the first lumen and the third lumen are separated except for the distal end portion, so each of these lumens can be used for different applications. For instance, a guide wire may be inserted in one of the lumens, while a contrast agent may be injected through the other lumen.

The forth aspect of the invention is a treatment instrument for endoscope which includes: an elongated insertion portion which is flexible, in which a lumen is formed; a first forceps piece disposed to rotate freely at a distal end of the insertion portion; a first conductive member connected to the first forceps piece and to a power supply device through the insertion portion; a distal end insertion portion which is fixed relative to the insertion portion, and which becomes the second forceps piece which receives the first forceps piece; in which the distal end insertion portion has a substantial cylindrical shape with a lumen; the distal end of the lumen opens to the distal end of the distal end insertion portion; the posterior end of the lumen opens to the outer surface different from the connecting surface connected with the insertion portion near the posterior end of the distal end insertion portion; and the distal end position of the distal lend insertion portion substantially coincides with the distal end position of the first forceps piece.

In the forth aspect of the treatment instrument for endoscope, a guide wire which is inserted in the lumen beforehand is passed through the conduit of the distal end insertion portion, and the distal end insertion portion is inserted in the entrance of the lumen along the guide wire. When inserting the distal end insertion portion in the entrance, the first forceps piece is kept open, the cut-out amount is adjusted by the insertion amount of the distal end insertion portion, and the cut-out position is adjusted by the direction of the first forceps piece. The biological tissue is held between the first forceps piece and the distal end treatment portion, high frequency electric current is applied, and the biological tissue is cut out. If necessary, the first forceps piece and the distal end insertion portion are inserted in the lumen from the cut out entrance. Since the distal end position of the distal end insertion portion coincides substantially with the distal end position of the first forceps piece, the cut-out amount can be adjusted by the insertion amount of the distal end insertion portion.

The fifth aspect of the invention is a treatment instrument for endoscope which includes: an elongated insertion portion which is flexible, in which a lumen is formed; a first forceps piece disposed to rotate freely at a distal end of the insertion portion; a first conductive member connected to the first forceps piece and to a power supply device through the insertion portion; a distal end insertion portion which becomes the second forceps piece which receives the first forceps piece; in which the distal end insertion portion has a substantial cylindrical shape; and the distal end position of the distal end insertion portion substantially coincides with the distal end position of the first forceps piece.

In the fifth aspect of the treatment instrument for endoscope, because the distal end position of the distal end insertion portion coincides with the distal end position of the first forceps piece, it is easy to adjust the cut-out length.

The sixth aspect of the invention is the treatment instrument for endoscope according to any of the first aspect of the invention to the fifth aspect of the invention, in which a reinforcement member for inhibiting the twist of the insertion portion around the axial line is inserted along the longitudinal direction of the insertion portion.

In the sixth aspect of the treatment instrument for endoscope, the reinforcement member can rotate the first forceps piece and the distal end insertion portion by transmitting the torque when the treatment instrument for endoscope is rotated around the axial line at the proximal side.

The seventh aspect of the invention is the treatment instrument for endoscope according to any of the first aspect of the invention to the fifth aspect of the invention, in which the first forceps piece has a cut-out electrode which protrudes while reducing its width toward the distal end insertion portion, the cut-out electrode is covered by insulation except for the distal end portion facing the distal end insertion portion, and the distal end insertion portion is insulated relative to the tissue and the cut-out electrode from which the first forceps piece is protruded.

In the seventh aspect of the treatment instrument for endoscope, the electric current density around the cut-out electrode increases, and the desired portion is cut out to a length substantially equal to the length of the cut-out electrode. Furthermore, the electric current density around the cut-out electrode is increased by insulating the distal end insertion portion.

The eighth aspect of the invention is the treatment instrument for endoscope according to any of the first aspect of the invention to the fifth aspect of the invention, in which the distal end insertion portion is made of a conductive material, and is configured such that it can be connected to a power supply device through a second conductive material provided in the insertion portion.

In the eighth aspect of the treatment instrument for endoscope, the treatment instrument for endoscope is a bipolar type cut-out forceps which cuts out the biological tissue by high frequency electric current flowing between the distal end insertion portion and the cut-out electrode. There is no need to install a return electrode in the body.

The ninth aspect of the invention is the treatment instrument for endoscope according to the eighth aspect of the invention, in which an electrode is formed by insulating the distal end insertion portion except the portion facing the first forceps piece.

In the ninth aspect of the treatment instrument for endoscope, the biological tissue is cut out by using a high frequency electric current which flows between the electrode of the distal end insertion portion and the cut-out electrode. The electric current density can be increased easily,

The tenth aspect of the invention is the treatment instrument for endoscope according to the ninth aspect of the invention, in which the electrode portion formed in the distal end insertion portion is elongatedly formed in the longitudinal direction of the distal end insertion portion to suit the cut-out electrode of the first forceps piece.

In the tenth aspect of the treatment instrument for endoscope, since the electrode of the distal end insertion portion is elongatedly formed, the electric current density is increased further, and the cut-out length or width to be cut out can be easily controlled

According to the present invention, the desired cut-out length and cut-out direction can be reliably obtained because the cut-out length is adjusted by the amount of insertion of the distal end insertion portion, the cut-out direction is adjusted by the direction of the first forceps piece, the adjusted cut-out line is fixed by holding it between the first forceps piece and the distal end insertion portion, and the cut-out by applying high frequency electric current is performed with the cut-out line in the held condition.

### BRIEF DESCRIPTION OF THE DRAWINGS

FIG. 1 shows a configuration of a treatment instrument for endoscope according to an embodiment of the present invention.
FIG. 2 is a view of FIG. 1 viewed from arrow A which shows an enlarged cut-away view of a portion of a distal end portion of the treatment instrument for endoscope.
FIG 3 is an enlarged view of the distal end portion of the treatment instrument for endoscope, which shows a first forceps piece in the open condition.
FIG. 4 is a cross sectional view along the line B-B of FIG 2.
FIG. 5 shows an example of an endoscope used together with the treatment instrument for endoscope.
FIG 6 is an explanatory diagram of a procedure for medical treatment showing the treatment portion protruded toward a duodenal papilla.
FIG 7 is a diagram showing a distal end insertion portion inserted in a duodenal papilla by opening the first forceps piece.
FIG 8 is a diagram showing an observation image of the endoscope. It is an explanatory diagram for adjusting an insertion amount of the distal end insertion portion and a direction of the first forceps piece.
FIG. 9 is a diagram showing a duodenal papilla held between the distal end insertion portion and the first forceps piece.
FIG. 10 is a diagram showing an observation image of the endoscope. It shows a duodenal papilla held between the distal end insertion portion and the first forceps piece.
FIG 11 is a diagram showing a duodenal papilla cut out by applying high frequency electric current.
FIG 12 is a diagram showing an observation image of the endoscope which shows a condition when the pre-cut of a duodenal papilla is completed.
FIG 13 is a diagram showing a treatment portion inserted in the bile duct through a cut out duodenal papilla.
FIG. 14 is a diagram showing a guide wire inserted in the bile duct through a lumen disposed in the treatment instrument for endoscope.
FIG. 15 is an explanatory diagram showing when the distal end insertion portion is inserted in a duodenal papilla after passing the guide wire through the bile duct beforehand.
FIG 16 is a partially sectional view showing the insertion portion provided with three lumens.
FIG 17 a diagram showing a configuration in which the distal end insertion portion is cut so as to make an insertion of the treatment portion easy.
FIG. 18 a diagram showing a curved portion formed beforehand in the distal end of the insertion portion.
FIG. 19 is an explanatory diagram showing an insertion portion having a curved portion being passed through the endoscope, and when it is made to protrude out of the endoscope.
FIG 20 is a diagram showing the distal end treatment portion inclined relative to the insertion portion.
FIG 21 is a diagram showing a configuration in which a first lumen directly exposes to the outside.
FIG 22 is a diagram showing an example of the use of the treatment instrument for endoscope shown in FIG. 21.
FIG 23 is a partially sectional view of a configuration of the distal end insertion portion which is offset.
FIG 24 is a diagram showing an example of the use of the treatment instrument for endoscope shown in FIG 23.
FIG. 25 is a diagram showing an example of the use of a single-lumen type insertion portion.
FIG 26 is a diagram showing a configuration of a bipolar type treatment instrument for endoscope.
FIG. 27 is a view of FIG 26 viewed from arrow C which shows a partially sectional enlarged view of a portion of the distal end portion of the treatment instrument for endoscope.
FIG 28 is a cross sectional view along the line D-D of FIG 27.
FIG 29 is a perspective view showing the first forceps piece in the open condition.
FIG 30 is a diagram showing a duodenal papilla being held between the distal end insertion portion and the first forceps piece.
FIG. 31 is a diagram schematically showing a flow of high frequency electric current.
FIG 32 is a diagram showing a duodenal papilla cut out by applying high frequency electric current.
FIG 33 is a diagram showing the guide wire inserted in the bile duct through the lumen in the treatment instrument for endoscope.
FIG. 34 is a perspective enlarged view showing the distal end portion of the treatment instrument for endoscope covered by insulation except for a portion of the distal end insertion portion.
FIG 35 is a diagram schematically showing a flow of high frequency electric current.
FIG. 36 is a diagram showing a configuration of a treatment instrument for endoscope having a cut-out wire.
FIG 37 is a cross sectional view along the line E-E of FIG. 36.
FIG. 38 is an explanatory diagram showing a procedure for additional cut-out using the cut-out wire.
FIG 39 is an explanatory diagram showing a disposition of a duodenal papilla and entrances of the bile duct and pancreatic duct.
FIG. 40 is a diagram showing a duodenal papilla being cut out.

### DETAILED DESCRIPTION OF THE INVENTION

Embodiments of the present invention will be described below in detail referring to the drawings. The same reference numerals are affixed to the same elements in each of the embodiments. Duplication of explanations is omitted.

### (First embodiment)

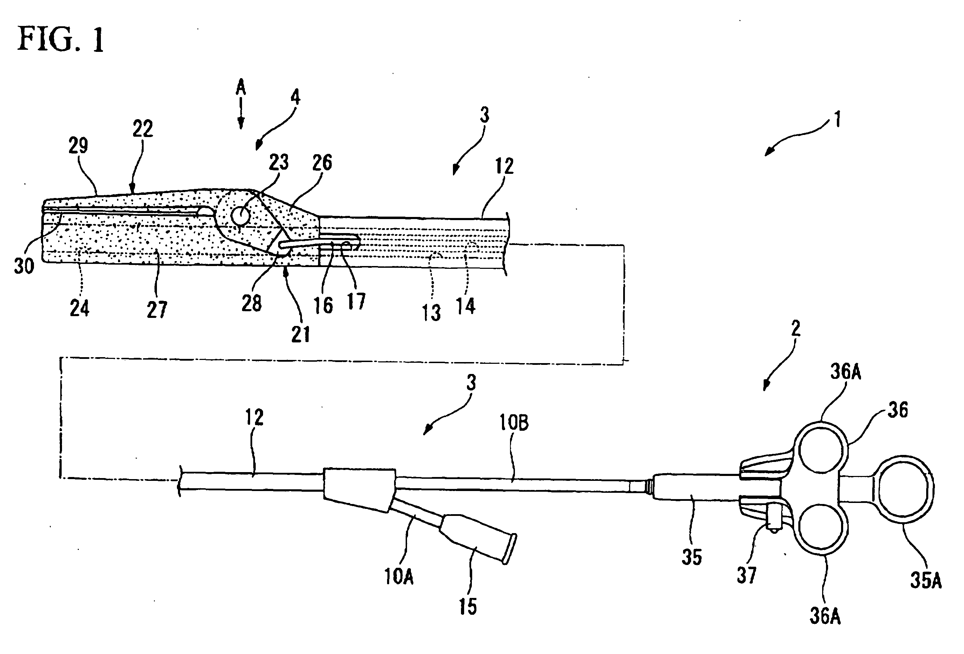

As shown in FIG 1, a treatment instrument for endoscope 1 (hereafter referred to as treatment instrument 1) is a monopolar dissecting forceps which includes an operation portion 2 that an operator operates, a flexible elongated insertion portion 3 which extends from the operation portion 2, and a treatment portion 4 provided at a distal end of the insertion portion 3.

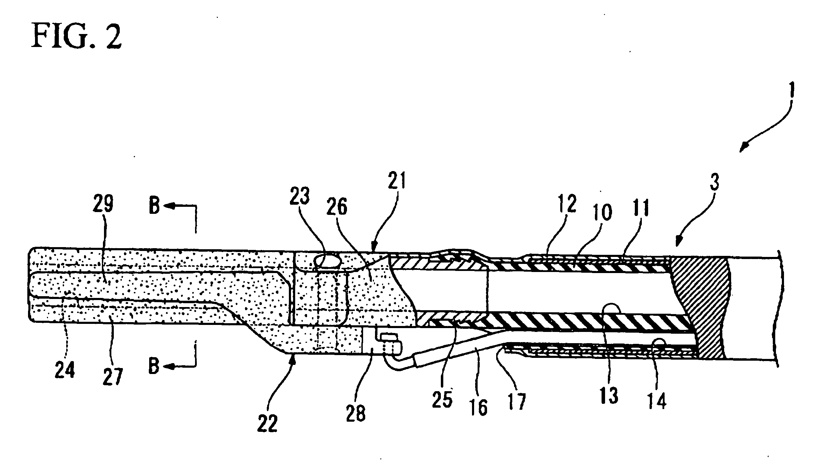

As shown in FIG. 1 and FIG. 2, the insertion portion 3 has a flexible sheath 10 thereinside, a braid 11 (reinforcing member), which is made of metallic mesh which transmits rotating torque and inhibits twist around the axial line, covers on the outer circumference of the sheath 10 and the braid is further covered by an insulating tube 12. The metallic mesh is made of stainless steel wire of diameter around 0.01 to 0.1 mm, for example, which is woven into lattice structure, or a mesh formed such that 2 to 12 of the wires are laid in parallel and bundled in a strip shape and 20 to 30 bundles of the strip may be woven into lattice structure. The braid 11 is not limited to metallic mesh, but any member with a specific strength may be used. Two lumens 13, 14 are formed in the sheath 10 substantially in parallel to the longitudinal direction of the sheath 10. The diameter of the first lumen 13 is of a size that allows a guide wire which is another treatment instrument or a contrast agent to pass through. The distal end portion of the first lumen 13 has an opening at the distal end of the insertion portion 3. At the proximal end portion of the sheath 10, a part which includes the first lumen 13 is branched at the proximal side. At the end portion of the branched sheath 10A, a cock 15 is placed through which a guide wire can be inserted or to which a syringe containing a contrast agent can be attached. The second lumen 14 has a smaller diameter than the first lumen 13. A distal end of the second lumen 14 has a slit-shaped opening at the side portion of the insertion portion 3. An operation wire 16 is passed through the second lumen 14 so as to freely advance and retract. A distal end of the operation wire 16 is drawn out from the distal end opening 17. A proximal end portion of the operation wire 16 is led into the operation portion 2. The operation wire 16 including the portion of the metallic wire such as stainless steel or steel wire drawn out from the distal end opening 17 is a first conductive member covered by an insulating material.

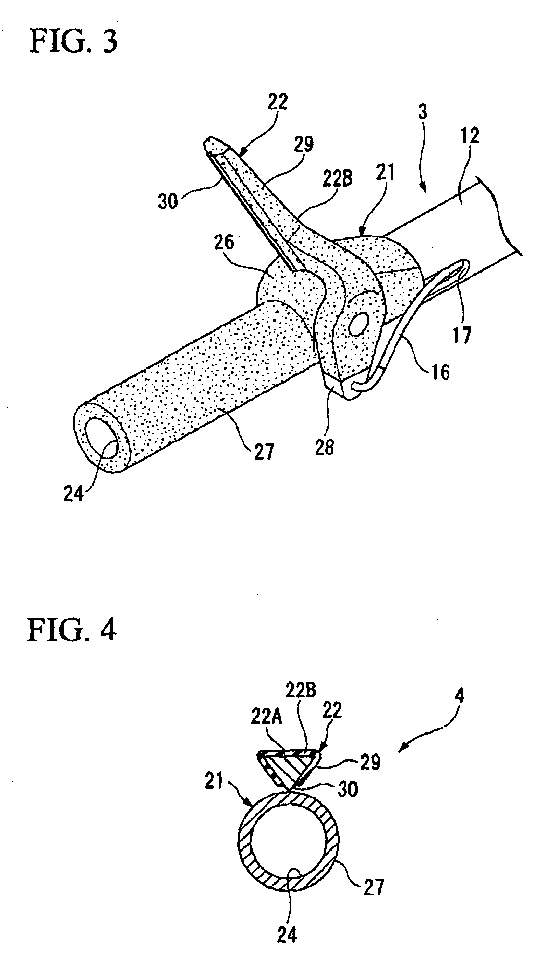

As shown in FIG 2 and FIG 3, the treatment portion 4 has a treatment portion body 21 fixed to the distal end of the insertion portion 3. A first forceps piece 22 is supported by pin 23 on the treatment portion body 21 so as to rotate freely. The treatment portion body 21 has a substantially cylindrical shape in which a lumen 24 (conduit) is formed.

The proximal end portion of the treatment portion body 21 is inserted in the first lumen 13 of the insertion portion 3, and a return 25 is provided in a protruding condition on its outer peripheral surface to prevent it from coming off.
Consequently, the lumen 24 of the treatment portion body 21 is coaxially formed with the first lumen 13 so as to be communicated. On a part of the insertion portion 3, in which the proximal end portion of the treatment portion body 21 is inserted, braid 11 is not provided. The braid 11 is provided substantially along the entire length of the insertion portion 3 excluding this part

A part of a portion of the treatment portion body 21, which is exposed to the distal end side than the insertion portion 3, protrudes outside in an expanded condition in the radial direction and forms a supporting portion 26. The pin 23 passes through the supporting portion 26 in an orthogonal direction to the lumen 24. A distal end insertion portion 27 with a substantially cylindrical shape with a constant diameter is formed on the distal end side of the treatment portion body 21 than the position at which the supporting portion 26 is formed. The diameter of the distal end insertion portion 27 is of a size such that it can be inserted in the papilla. However, if the object is to pass a guide wire of the most common size of 0.035 inch (0.89 mm) through the lumen 24, the lumen diameter becomes around 0.9 to 1.0 mm, and the diameter of the distal end insertion portion 27 becomes around 1.4 to 2.0 mm. Since the diameter of a general treatment instrument is around 2 mm, the above thickness is sufficiently thin as a diameter.

Also, when insertion into the bile duct is difficult, it may be easy to insert a treatment instrument, a distal end of which formed thin. Following the example, if improving insertability into papilla is prioritized by making the diameter of the distal end insertion portion 27 as small as possible, an inner diameter of the lumen 24 can be made as small as around 0.3 mm by restricting its usage only to injection of the contrast agent, and as a result, the outer diameter of the distal end insertion portion 27 can be made smaller than 1 mm.

The length of the distal end insertion portion 27 is, for example, 3 to 10 mm. The length of the distal end insertion portion 27 should be at least around 3 mm in order to rotate the first forceps piece 22 around the distal end insertion portion 27 as an axis. If the length is shorter than 3 mm, then it is more likely to come off during rotation and operation may become more troublesome. Also, if the distal end insertion portion 27 is too long, then its inducement into the bile duct, which directs toward the mouth side (upward) of the duodenum, becomes difficult, and since its strength in holding the tissue together with the first forceps piece 22 becomes weaker, the length around 10 mm is appropriate at the maximum. Within the length of 3 to 10 mm, 5 to 7 mm is even more optimum for the length of the distal end insertion portion 27. Considering the ease of the inducement into the bile duct, shorter length is preferable as the length of distal end insertion portion 27; however, the general cut-out length for treatment of the bile duct is 5 to 10 mm. Thus, if the distal end insertion portion 27 is short, the number of cut-outs increases, and the labor increases. Accordingly, considering a balance between the ease of inducement into the bile duct and the number of cut-outs, 5 to 7 mm is more preferable.

The distal end opening of the lumen 24 is formed on the distal end surface of the distal end insertion portion 27. An axial line of the lumen 24 and the longitudinal direction of the distal end insertion portion 27 are substantially parallel to the axial line, that is the longitudinal direction, of the insertion portion 3.

The treatment portion body 21 may be made of ceramic such as alumina or zirconia, or metallic material processed such that the outer periphery of which insulating coating, or made of fluororesins such as polytetrafluoroethylene (PTFE) or ethylene-tetrafluoroethylene copolymer) ETFE, or high heat resistant and high hardness resins or resin elastomers such as polyetheretherketone (PEEK). Ceramic coating or resin coatings such as PTFE coating are cited as examples of the insulating coating.

A proximal end side of first forceps piece 22 than a part pivotally supported by the pin 23 extends toward the side of the treatment portion body 21 and forms a linking portion 28, The operation wire 16 drawn from the insertion portion 3 is connected to the linking portion 28. The distal end side of the first forceps piece 22 than the pin 23 is curved so as to avoid the supporting portion 26, and forms a forceps portion 29 which extends substantially in parallel to the axial line of the distal end insertion portion 27. The length of the forceps portion 29 is substantially the same as the length of the distal end insertion portion 27. The distal end position of the forceps portion 29 substantially coincides with the position of the distal end surface of the distal end insertion portion 27. As shown in FIG 3 and FIG 4, the forceps portion 29 has a triangular cross sectional shape. The first forceps piece 22 is processed by coating the main body 22A made of a conductive material with insulating layer 22B. The insulating layer 22B covers the entire first forceps piece 22 except for the linking portion 28 through which the operation wire 16 passes, and one of the vertexes portions (cut-out electrode 30) of the triangular shape of the forceps portion 29. An electrical connection with a metallic wire of the operation wire 16 is formed in the linking portion 28. The cut-out electrode 30 is the vertex portion facing the distal end insertion portion 27. The width of the cut-out electrode 30 is sufficiently thin, for example less than 0.3 mm, so that electric current density is increased. The insulating layer 22B is made of a resin with insulating properties such as PTFE, ETFE, or PEEK, or it may be made of an insulating material such as ceramics like alumina or zirconia, or diamond like carbon (DLC), or diamond like nanocomposite (DLN).

In such a treatment portion 4, when the operation wire 16 is advanced or retracted, the first forceps piece 22 rotates relative to the treatment portion body 21, and the open condition shown in FIG. 3 can be changed to the closed condition shown in FIG. 1. In the closed condition, the biological tissue can be held between the distal end insertion portion 27 and the first forceps piece 22. For this reason, the distal end insertion portion 27 functions as a second forceps piece which works together with the first forceps piece 22, when holding the biological tissue.

As shown in FIG 1, the operation portion 2 is connected to a proximal end of the branched sheath 10B at the insertion portion 3, and it has an elongated operation portion body 35 which extends by substantially coinciding with the axial line of the sheath 10B. A ring 35A for hooking fingers is provided at the distal end of the operation portion body 35. A slider 36 is disposed so as to freely advance and retract to the distal end side than the ring 35A. A ring 36A for hooking fingers is also provided in the slider 36. The proximal end portion of the operation wire 16 is fixed to the slider 36. The operation wire 16 is electrically connected to the electric connector 37 which is provided in the slider 36 in a protruding condition. The electric connector 37 has a shape which enables connection to the high frequency power supply which is not shown in the figures.

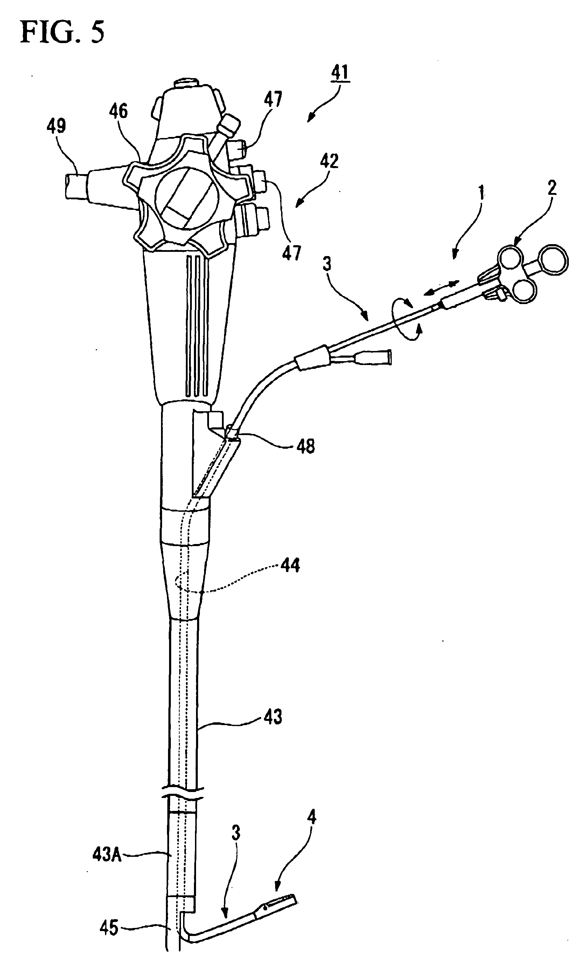

FIG 5 shows the endoscope 41 used together with the treatment instrument. The endoscope 41 includes an endoscope operation portion 42 which is used by an operator held in his hand, and an endoscope insertion portion 43 extending from the endoscope operation portion 42 and inserted in the body. The endoscope insertion portion 43 is elongated and flexible, with a working channel 44 through which treatment instrument 1 or other treatment instrument passes, a channel for delivering air and liquid (not shown in the figures), and so on formed thereinside. A cover 45 is attached to a distal end of the endoscope insertion portion 43. A distal end opening of each kind of channel, imaging device, and lighting device are disposed in the cover 45. Since the cover 45 is a side-view type cover, the imaging device and the lighting device are disposed such that their lines of sight are directed laterally. Also, an elevator (not shown in the figures) for changing the direction of the treatment instrument 1 is provided in the cover 45.

Angle knob 46 and various kinds of switches 47 are disposed in the endoscope operation portion 42. If the angle knob 46 is operated, the angled portion 43A at the distal end of the endoscope insertion portion 43 can be operated to be curved in the desired direction. A forceps plug 48 is provided at the side portion of the endoscope operation portion 42. The forceps plug 48 blocks the opening of the working channel 44. Also, the forceps plug 48 has a slit formed therein such that the treatment instrument 1 can be inserted in the working channel 44 therethrough. A universal cable 49 extends from the endoscope operation portion 42, and is connected to a control device which is not shown in the figures. The control device processes the images taken by the imaging device to display them on a monitor, and controls the air and liquid delivery device and the lighting device.

Next, operations of the embodiment are described hereafter. Medical treatment performed on the bile duct, which is a lumen, is described hereafter, but medical treatment performed on other locations under other objectives is applied by the embodiment.

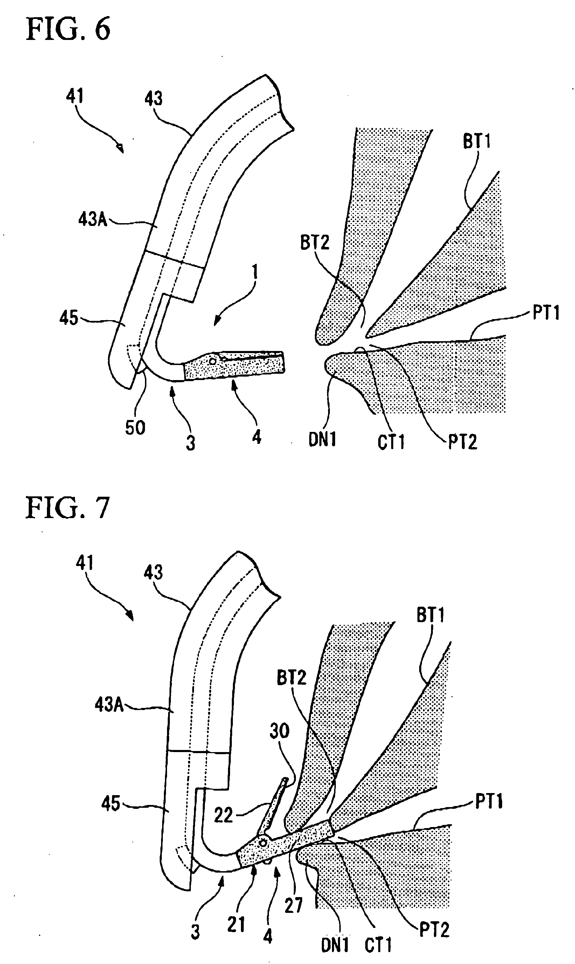

First, the endoscope insertion portion 43 is inserted from the patient's mouth, and the cover 45, disposed at distal end thereof, is delivered to the vicinity of the duodenal papilla while monitoring the imaging device. Next, the treatment instrument 1 is delivered to the working channel 44 by passing through the forceps plug 48. As shown in FIG 6, the distal end portion of the treatment instrument 1 is curved toward the duodenal papilla DN1 using the elevator 50 provided in the cover 45. As shown in FIG 2, the braid 11 and the proximal end of the treatment portion 4 do not overlap at the distal end of the insertion portion 3, whereby the distal end portion of the insertion portion 3 can be easily curved.

The slider 36 of the treatment instrument 1 is advanced, and the first forceps piece 22 linked to the operation wire 16 is rotated and opened. In this condition, the entire treatment instrument 1 is advanced, and the distal end insertion portion 27 is inserted in the duodenal papilla DN1, as shown in FIG 7. A syringe (not shown in the figures) is attached to the cock 15 at the proximal end of the sheath 10A, by passing through the first lumen 13, contrast agent is injected in the common channel CT1, the bile duct BT1, or the pancreatic duct PT1 in the duodenal papilla DN1, then the position and the inserted amount of the distal end insertion portion 27 can be confirmed by X-ray photography. Although the distal end insertion portion 27 is expected to be inserted up to the bile duct BT1, when it is difficult to insert in the bile duct BT1, it may be inserted up to the common channel CT1. If the common channel CT1 is short, and the insertion length of the distal end insertion portion 27 is too short for rotating operation thereafter, then the distal end insertion portion 27 is inserted a bit into the pancreatic duct PT1. However, to minimize damage to the pancreatic duct PT1 during cut-out, the insertion into the pancreatic duct PT1 should preferably be restricted to a few mm. Thus, it should be inserted while confirming the difference in the overall length of the distal end insertion portion 27 and the length of the portion exposed outside from the papilla DN1 by the endoscopic images, or while observing the inserted length of the distal end insertion portion 27 by X-ray images.

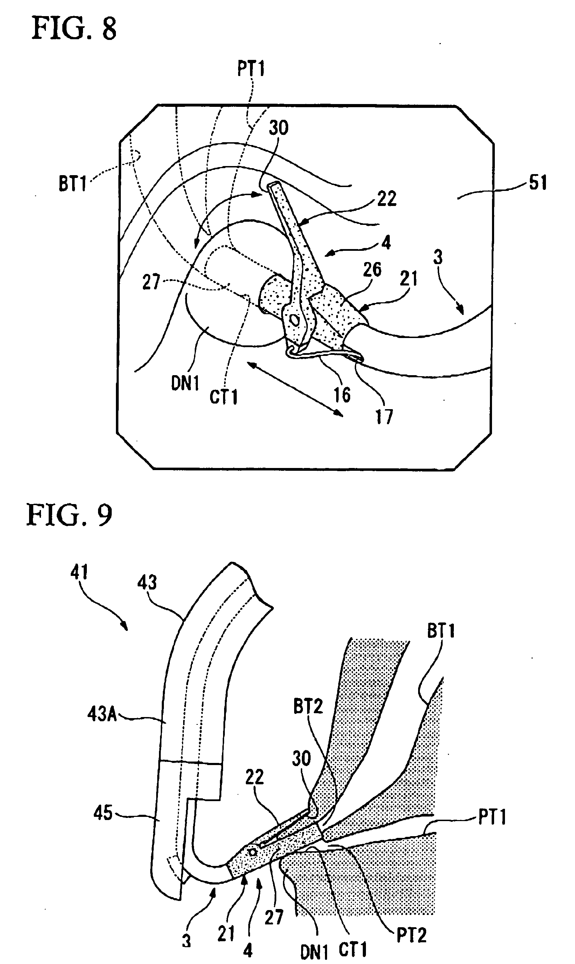

As shown by arrows in FIG. 5, the operation portion 2 or the insertion portion 3 is rotated around the axial line, and the direction of the first forceps piece 22 is adjusted to a desired direction. Since the metallic braid 11 is inserted in the insertion portion 3, the rotating torque input from the proximal side can be transmitted easily to the distal end. As a result, the first forceps piece 22 rotates about the axial line of the insertion portion 3 and the treatment portion body 21. As shown in FIG 8, the operator checks the position of the first forceps piece 22 by the image 51 photographed by the observation device of the endoscope 41. If treatment is to be taken relative to the bile duct BT1, and if the cutting out direction is taken as the 12 o'clock direction for the mouth side of the duodenum and the six o'clock direction for the anus side, then cut-out should preferably be performed in the 11 to 12 o'clock direction along which the bile duct HT1 extends. Therefore, the treatment instrument 1 is rotated so that the first forceps piece 22 is disposed at this position. Since the insertion length of the distal end insertion portion 27 may be offset during the rotating operation of the first forceps piece 22, endoscopic images (confirm the insertion length from the difference between the overall length of the distal end insertion portion 27 and a length of a portion exposed outside from the papilla DN1) or X-ray images is used to confirm the insertion length of the distal end insertion portion 27, and the insertion length is corrected if necessary.

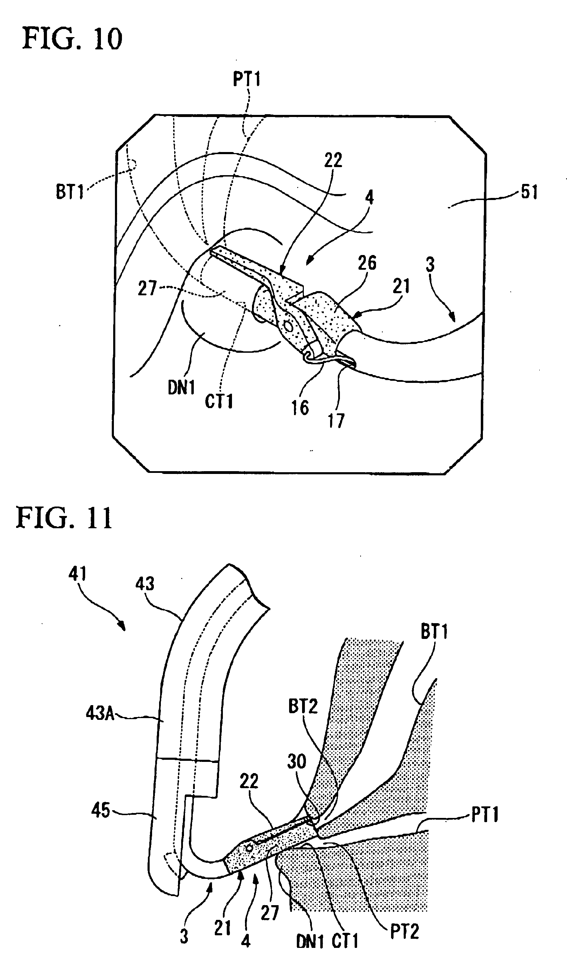

Next, the slider 26 is retracted and the first forceps piece 22 is closed. As shown in FIG 9, the duodenal papilla DN1 is held between the cut-out electrode 30 of the first forceps piece 22 and the distal end insertion portion 27. A linear portion held in this way becomes the cut-out line. If a high frequency electric current is applied subsequently, a portion held between the pair of forceps piece (the first forceps piece 22 and the distal end insertion portion 27) will be cut out, but as shown in FIG 10, by endoscopic observations, the portion held by the first forceps piece 22, that is, the cut-out position and the cut-out length should be judged if or not appropriate before cutting out. If judged to be inappropriate, the rotation and advancement and retraction of the treatment instrument 1 are performed after opening the first forceps piece 22, and after re-adjusting to the appropriate position, the first forceps piece 22 is closed. Instead of endoscopic observations, or in addition to the endoscopic observations, X-ray observations may be performed and the cut-out position or cut-out length may be confirmed.

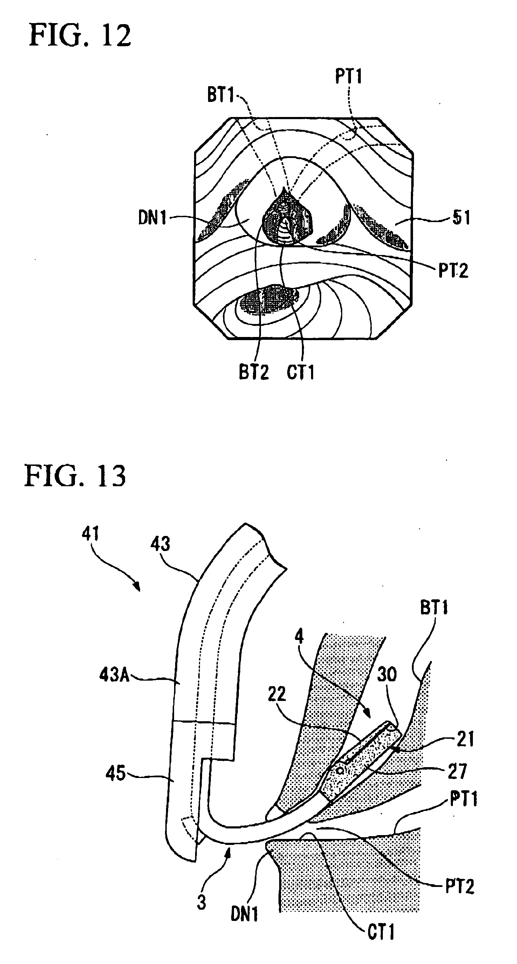

A high frequency power supply is connected to the electric connector 37 of the operation portion 2, and high frequency electric current of mixed waveforms having the properties of the cut-out waveform or having both of the properties of cut-out and coagulation is made to flow to the first forceps piece 22 through the operation wire 16. The high frequency electric current flows from the first forceps piece 22 to the portion to which the cut-out electrode 30 of the duodenal papilla DN1 is contacted, and is returned to the high frequency power supply after passing through the return electrode which is contacted to the patient. Since portions other than the cut-out electrode 30 or the treatment portion body 21 are insulated, the high frequency electric current is not passed through these portions. Since the cut-out electrode 30 becomes gradually narrower toward the distal end insertion portion 27, concentrated high frequency electric current flows to the duodenal papilla DN1 held between the first forceps piece 22 and the distal end insertion portion 27, the electric current density increases, and the tissue is cut out. As a result, the duodenal papilla DN1 is cut out, as shown in FIG 11. If the cut-out amount is judged inadequate from endoscopic observations, the operation described above is repeated to increase the cut-out amount. When the duodenal papilla DN1 is cut out, as shown in FIG. 12, the entrance BT2 of the bile duct BT1 and the entrance PT2 of the pancreatic duct PT1 can be separated, the entrance BT2 of the bile duct BT1 can be confirmed by endoscopic observations, and the treatment instrument 1 can be easily inserted in the bile duct BT1.

When cut-out is performed with the distal end insertion portion 27 inserted in the bile duct BT1, the cut-out length is generally more than adequate even for retrieval of gallstones, but if cut-out is performed with the insertion portion inserted up to the common channel CT1 or slightly inserted in the pancreatic duct PT1, the cut-out length at the exit of the bile duct BT1 is generally inadequate and the opening is smaller than required. Accordingly, the distal end insertion portion 27 is inserted in the separated bile duct mouth BT2 after opening the first forceps piece 22. Then the cut-out length and the cut-out direction are adjusted again, the cut-out portion is held, current is applied, and additional cut-out is performed.

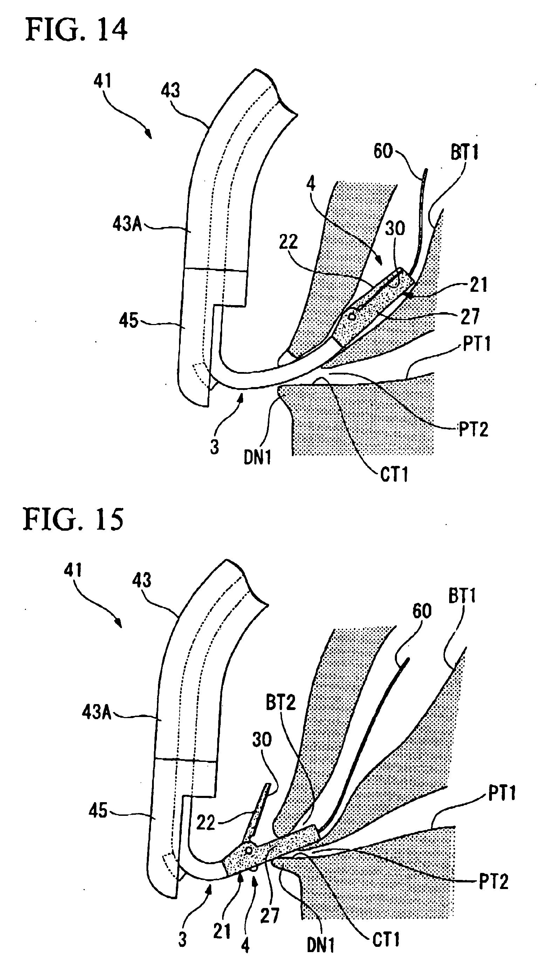

When adequate cut-out length is obtained, the operation portion 2 is operated and the first forceps piece 22 is closed, and the treatment portion 4 is inserted in the bile duct BT1, As shown in FIG. 13, when the treatment portion 4 is inserted in the bile duct BT1, the contrast agent may be re-sent if necessary, and the inside of the bile duct BT1 is imaged. The syringe containing the contrast agent is removed from the cock 15 and, instead, the guide wire is inserted in the first lumen 13. As shown in FIG 14, the guide wire 60 is inserted to the bile duct BT1 by passing through from the first lumen 13 (see FIG. 2) to the lumen 24 of the treatment portion 4 (see FIG. 2). When replacing the treatment instrument, the guide wire 60 is remained as it is and only the treatment instrument 1 is pulled out, and another treatment instrument is delivered to the bile duct BT1 by using the guide wire 60 as a guide. Other treatment instruments include forceps or balloons or biopsy forceps provided with cups for biopsy for pulverizing or retrieving gallstones and the like in the bile duct. When all the required medical treatments are completed, other treatment instruments, the guide wire 60, and the endoscope 41 are each pulled out of the body.

Also, before inserting the treatment instrument 1, if the guide wire 60 is already inserted from the duodenal papilla DN1 into the bile duct BT1, then the distal end insertion portion 27 and the treatment portion 4 may be delivered to the duodenal papilla DN1 by following the guide wire 60, as shown in FIG. 15. In this manner, the distal end insertion portion 27 can be easily inserted in the duodenal papilla DN1 and the bile duct BT1 from the endoscope 41.

According to the present invention, the desired cut-out length and cut-out direction can be reliably obtained since the cut-out length and the cut-out direction are adjusted with the distal end insertion portion 27 in the inserted condition in the duodenal papilla DN1, the adjusted cut-out line is fixed by being held between the first forceps piece 22 and the distal end insertion portion 27, and high frequency electric current is applied in the held condition to perform the cut-out. Even if the held portion is not the desired cut-out length and not in the desired cut-out direction, the first forceps piece 22 can be opened and re-adjusted before cutting out. Thus, inappropriate cut-out direction can be reliably avoided, and the medical treatment can be easily and reliably performed.

Since the distal end position of the distal end insertion portion 27 substantially coincides with the distal end position of the first forceps piece 22, cut-out length same as the inserted length of the distal end insertion portion 27 can be obtained. Therefore, if the distal end insertion portion 27 is inserted up to the end of the common channel CT1, or up to entrance of the pancreatic duct PT1, the entrances BT2 and PT2 of the bile duct BT1 and the pancreatic duct PT1 respectively can be separated (pre-cut) by the cut-out. In this way, even performing pre-cut, which was difficult in the past, desired cut-out length and cut-out direction can be easily and reliably obtained.

Also, the cut-out length in the lumen, which cannot be directly seen in the endoscopic images coincides with the inserted length of the distal end insertion portion 27. Therefore, by checking the length inserted in the lumen of the distal end insertion portion 27 by X-rays, or by estimating the insertion length from the difference between the overall length of the distal end insertion portion 27 and the length of the exposed portion outside the lumen, the length can be known before the cut-out, accordingly a cut-out length longer than the desired length can be prevented.

By making the distal end insertion portion 27 in substantially cylindrical shape, it can be easily rotated while inserted in the duodenal papilla DN1, and the cut-out position can be easily adjusted. The elongated insertion portion 3 is made to easily transmit the rotating torque through the metallic braid 11 or through the operation wire 16. Thus, the operation by the operator can be reflected more accurately. The cut-out length can be controlled by the insertion length of the distal end insertion portion 27 or the number of cut-outs. The insertion length can be easily adjusted by the amount of advancement and retraction of the treatment instrument 1. By using the side-view type endoscope 41, the insertion length or the cut-out position can be checked easily, but the endoscope 41 is not limited to the side-view type in special case studies such as studies after surgical operations.

A lumen 24 communicating to the first lumen 13 is provided in the treatment portion body 21, so the common channel CT1, the pancreatic duct PT1 or the bile duct BT1, can be imaged without inserting the entire treatment portion 4.

If the guide wire 60 is introduced through the lumen 24, the treatment instrument can be easily replaced, and the efficiency of the procedure improves.

Since adequate hardness is provided in the treatment portion body 21 so as to receive the first forceps piece 22, the tissue can be firmly held. One of the vertexes of the first forceps piece 22 is made as the cut-out electrode 30 and the electric current density is made to be increased so that the biological tissue can be reliably cut out.

The distal end insertion portion 27 is of substantially cylindrical shape and the shape is not incisive, therefore, even if bleeding occurs due to cut-out, the bleeding portion can be held by the first forceps piece 22 and the distal end insertion portion 27 without applying current, pressure can be applied on the bleeding portion, and bleeding can be stopped. Or, to the bleeding portion held as described above, a high frequency electric current for coagulation instead of a high frequency electric current for cut-out can be applied and the bleeding is stopped by coagulation without cutting out. As a result, bleeding can be stopped immediately without replacing the treatment instrument 1.

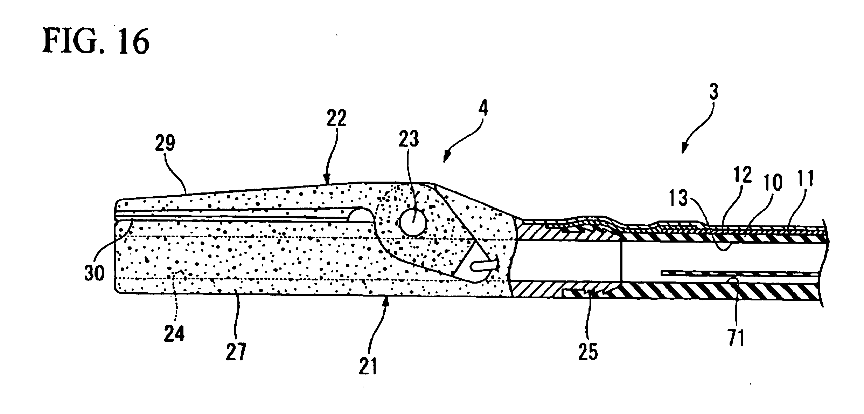

As in the treatment instrument shown in FIG 16, a third lumen 71 may be added in addition to the first and second lumens 13, 14 in the sheath 10 of the insertion portion 3. The first lumen 13 and the third lumen 71 merge at the distal end and are both communicated to the lumen 24 of the treatment portion body 21. The guide wire 60 and other treatment instruments are passed through the first lumen 13. The third lumen 71 has a smaller cross section than the first lumen 13, and the contrast agent is injected therein. Both of these are passed through the lumen 24 of the treatment portion body 21. The first and third lumens 13 and 71 branch on the operation portion 2 side and a cock is attached in each lumen.

The procedure for medical treatment using the treatment instrument 1 is similar to the procedure mentioned above. Since the path (the first lumen 13) for passing through mainly the guide wire 60 and the like, and the path (the third lumen 71) for passing through mainly the contrast agent are different, and the contrast agent with high viscosity and the guide wire 60 do not come in contact with each other directly, the guide wire 60 can be pulled out or inserted smoothly.

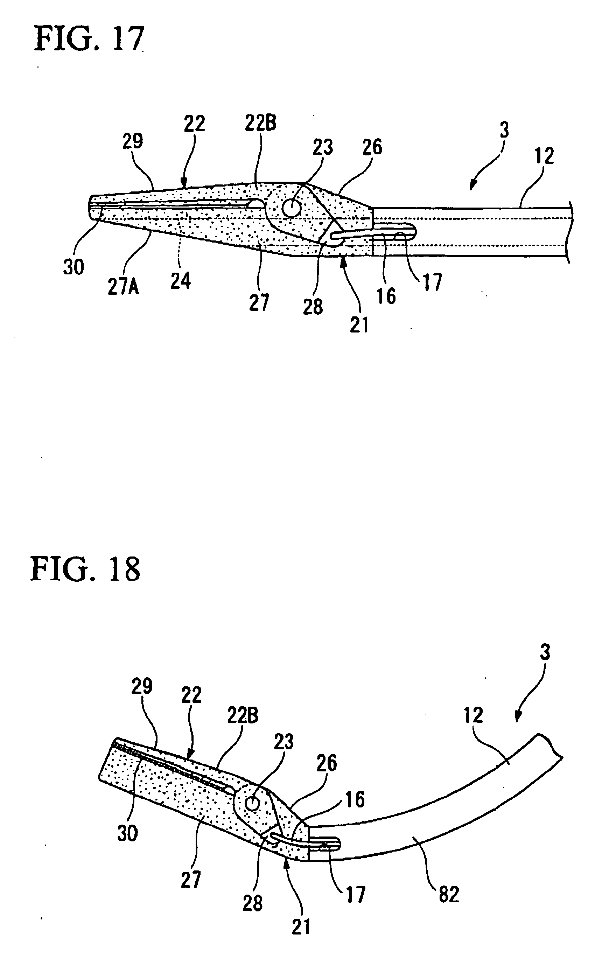

Also, as shown in FIG 17, the distal end insertion portion 27 may be cut diagonally so that its outer shape becomes smaller as closer to the distal end. An end face 27A formed by the cut is provided on the opposite side from the first forceps piece 22 side. Since the distal end insertion portion 27 becomes small, it can be easily inserted in the duodenal papilla DN1. The treatment portion 4 can be smoothly inserted in the bile duct BT1 and the like since the distal end side becomes small for the entire treatment portion 4 also.

### (Second embodiment)

As shown in FIG 18, the treatment instrument for endoscope 81 (hereafter referred to as treatment instrument 81) is a monopolar cut-out forceps provided with a curved portion 82 having a curved accent in the distal end of the insertion portion 3. The curved accent is made by performing a heat treatment on a curved wire, through which the insertion portion 3 passed, in the production stage. The curved portion 82 is curved in the direction in which the first forceps piece 22 opens.

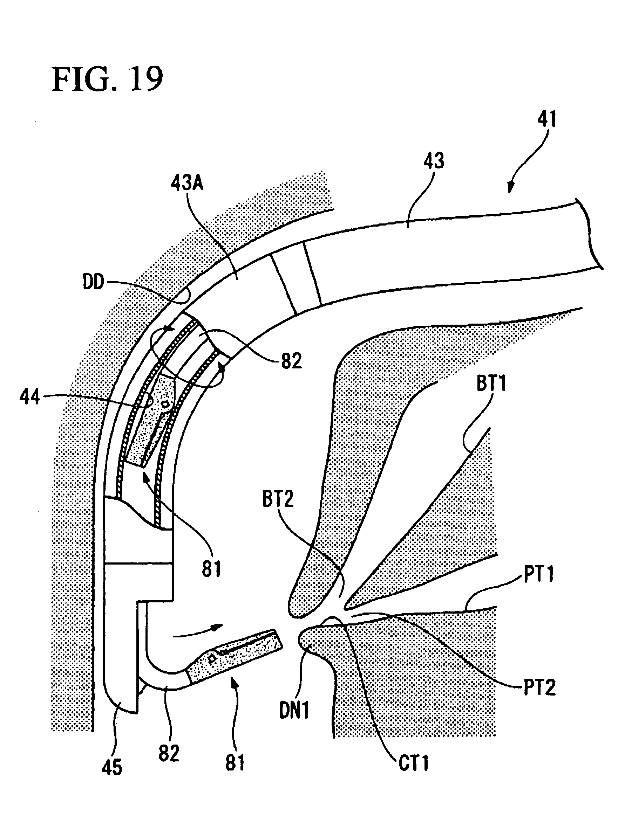

As shown in FIG. 19, the angled portion 43A of the endoscope 41 is curved along the curve DD from a stomach to a duodenum. Even if the direction of the first forceps piece 22 faces an arbitrary direction when the treatment instrument 81 is inserted in the working channel 44, when the curved portion 82 of the treatment instrument 81 passes through the curved portion of the angled portion 43A, the curved portion 82 follows the curved shape of the angled portion 43A and rotates the insertion portion 3 and the treatment portion 4, thus the first forceps piece 22 is positioned naturally on the inside of the curve. Accordingly, when the treatment portion 4 protrudes from the distal end cover 45 of the endoscope 41, the first forceps piece 22 faces substantially upward relative to the duodenum DD, that is, it comes out facing a direction in which the duodenal papilla DN1 is to be cut out, accordingly the subsequent positioning work can be reduced.

Also, since the direction of the curved portion 82 is that the: treatment portion 4 is directed toward the duodenal papilla DN1, its insertion into the duodenal papilla DN1 also becomes easy.

The distal end insertion portion 27 is of substantially cylindrical shape and the shape is not incisive, therefore, even if bleeding occurs due to cut-out, the bleeding portion can be held by the first forceps piece 22 and the distal end insertion portion 27 without applying current, pressure can be applied on the bleeding portion, and bleeding can be stopped. Or, with the bleeding portion held as described above, a high frequency electric current for coagulation instead of a high frequency electric current for cut-out can be applied, and the bleeding is stopped by coagulation without cutting out.

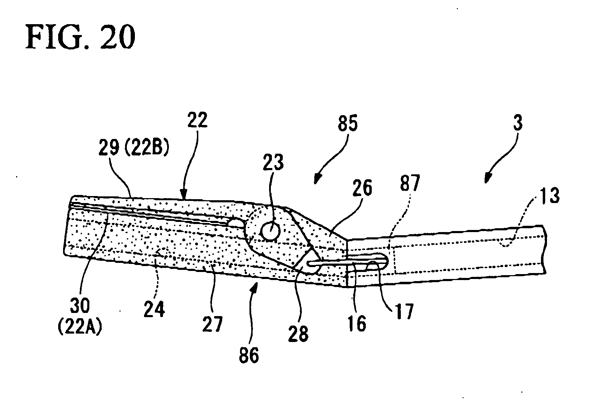

The treatment portion body 86 may be inclined relative to the axial line of the insertion portion 3 as in the treatment portion 85 shown in the modified example in FIG. 20. The treatment portion 85 is provided with a bent portion 87 in which the proximal end portion of the treatment portion body 86 is inclined relative to the axial line of the treatment portion body 86, and the bent portion 87 is pushed into the first lumen 13 to be fixed therein. The direction of inclination of the treatment portion 85 relative to the axial line of the insertion portion 3, that is, the direction of inclination of the distal end insertion portion 27 is such that the first forceps piece 22 side is raised, and its angle of inclination may be 10 degrees for example. In a case of using this treatment instrument as well, when passing through the curved portion of the angled portion 43A of the endoscope 41, the bent portion 87 follows the curved shape of the angled portion 43A, and rotates the insertion portion 3 and the treatment portion 4; therefore, the first forceps piece 22 comes out in the direction in which the duodenal papilla DN1 is to be cut out. Moreover, since the direction of inclination of the treatment portion 85 is disposed so as to face the duodenal papilla DN1, it is easy to insert the distal end insertion portion 27 in the duodenal papilla DN1.

### (Third embodiment)

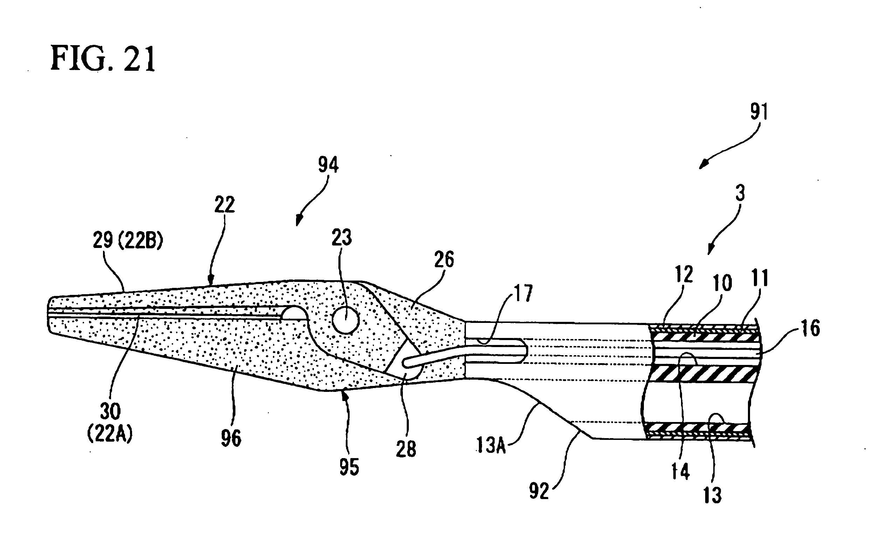

As shown in FIG. 21, the treatment instrument for endoscope 91 (hereafter referred to as "treatment instrument 91 ") is a monopolar cut-out forceps wherein insertion portion 92 has a sheath 10, which includes lumens 13 and 14, is covered by a metallic braid 11 and insulating tube 12. The distal end portion of the insertion portion 3 has an end face 92 cut diagonally so that it becomes thin closer to the distal end thereof and the distal end opening 13A of the first lumen 13 is provided therein. That is, the first lumen 13 is exposed directly outside from the distal end of the insertion portion 3. At the distal end of the second lumen 14, a distal end opening 17 is formed at the distal end side portion of the insertion portion 3 and the operation wire 16 is drawn out therefrom toward the treatment portion 94.

The treatment portion 94 is constituted such that the treatment portion body 95 is fixed substantially coaxially with the axis of the second lumen 14. The treatment portion body 95 has a supporting portion 26 which pivotally supports the first forceps piece 22 by the pin 23 and distal end side than the supporting portion 26 forms a distal end insertion portion 96 which is a second forceps piece as well. The distal end insertion portion 96 extends substantially parallel to the axial line of the insertion portion 92. The distal end insertion portion 96 can be inserted in the duodenal papilla DN1, and has an outer shape which reduces (for example, up to 1 mm or less) closer to the distal end. The length of the distal end insertion portion 96 is 3 mm to 10 mm which is similar to the first embodiment, and more preferably 5 mm and 7 mm. The distal end position of the distal end insertion portion 96 and the distal end position of the first forceps piece 22 are substantially the same. The material which forms the treatment portion 94 is the same as that of the first embodiment.

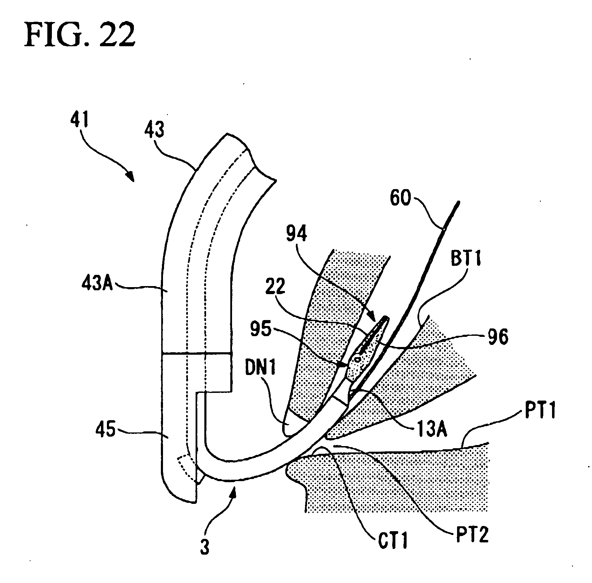

During medical treatment, the treatment instrument 91 is made to protrude from the endoscope 41, and the distal end insertion portion 96 is inserted in the duodenal papilla DN1. After adjusting the insertion amount and the direction of the treatment portion 94, the portion held between the first forceps piece 22 and the distal end insertion portion 96 is cut out by high frequency electric current. As shown in FIG. 22, the treatment portion 94 is inserted in the bile duct BT1 from the duodenal papilla DN1 the opening of which is extended by the cut-out. After insertion up to the distal end opening 13A in the bile duct BT1, contrast agent is injected into the bile duct BT1 from the first lumen 13 to perform X-ray observation, or guide wire 60 is passed through. When the guide wire 60 is passed through, the treatment instrument 91 is pulled out of the body while detaining the guide wire 60. Another treatment instrument, such as a balloon or biopsy forceps is inserted in the bile duct BT1 from the endoscope 41 along the guide wire 60, and necessary treatments are performed.

According to the present embodiment, the first lumen 13 is provided in a offset position relative to the treatment portion 94, and treatment portion 94 is constituted so that no lumen is formed therein, the treatment portion 94 can be made thinner. As a result, the treatment portion 94 can be easily inserted in the bile duct BT1 or the pancreatic duct PT1 from the duodenal papilla DN1. Moreover, since the distal end insertion portion 96 becomes thinner, the distal end insertion portion 96 can be easily inserted in the duodenal papilla DN1, the bile duct BT1, or the pancreatic duct PT1, before the cut-out. The distal end insertion portion 96 is of substantially cylindrical shape and the shape is not incisive, therefore, even if bleeding occurs due to cut-out, the bleeding portion can be held by the first forceps piece 22 and the distal end insertion portion 96 without applying current, pressure can be applied on the bleeding portion, and bleeding can be stopped. Or, with the bleeding portion held as described above, a high frequency electric current for coagulation instead of a high frequency electric current for cut-out is applied and the bleeding can be stopped by coagulation without cut-out. The effects of cut-out are the same as in the first embodiment.

### (Fourth embodiment)

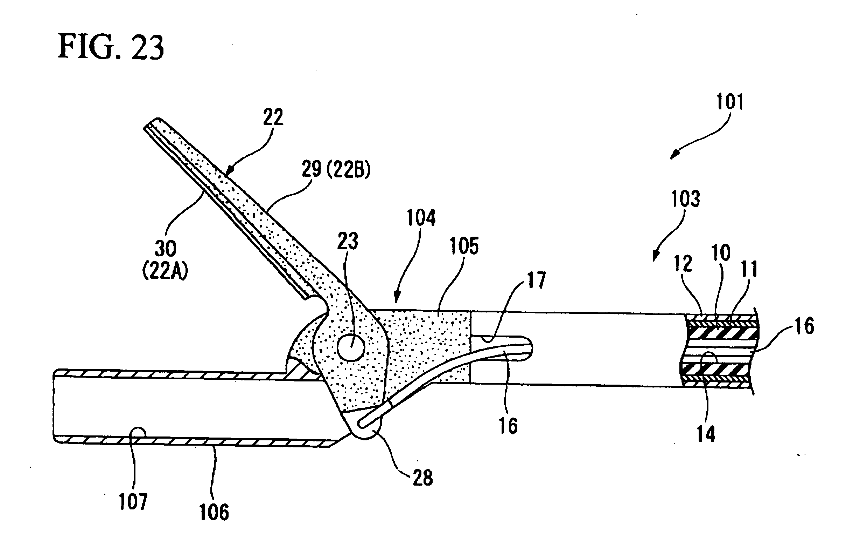

As shown in FIG 23, the treatment instrument for endoscope 101 (hereafter referred to as treatment instrument 101) is a monopolar cut-out forceps provided with a single-lumen type insertion portion 103. The insertion portion 103 is provided with a sheath 10 having a lumen 14 therein and laminated with a braid 11 and an insulating tube 12. The distal end opening 17 of the lumen 14 is formed on the side portion of the distal end of the insertion portion 103. An operation wire 16 is passed through the lumen 14 so as to freely advance or retract therein.

The treatment portion 104 has a treatment portion body 105 which extends on a line extending from the axial line of the insertion portion 103, in which a first forceps piece 22 is attached to the treatment portion body 105 by the pin 23 so as to rotate freely. From the distal end of the treatment portion body 105, a distal end insertion portion 106 which is substantial cylindrical shape extends in offset manner relative to the axial line thereof. The distal end insertion portion 106 becomes a receiving portion which receives the cut-out electrode 30 of the first forceps piece 22, and also becomes a second forceps piece working together with the first forceps piece 22 to hold the duodenal papilla DN1. The distal end insertion portion 106 is integrally fixed with the treatment portion body 105 such that it extends substantially parallel to the axial line of the treatment portion 104, and it has a substantially cylindrical shape formed with a lumen 107 which passes through from the distal end to the proximal end thereof. The distal end opening of the lumen 107 opens out to the distal end of the distal end insertion portion 106. The posterior end opening of the lumen 107 opens out to an outer surface which is different from the connecting surface connected to the insertion portion 103 close to the posterior end of the distal end insertion portion. The length of the distal end insertion portion 106 is 3 mm to 10 mm, similar to the first embodiment; more preferably 5 mm to 7 mm. The distal end position of the distal end insertion portion 106 and the distal end position of the first forceps piece 22 are substantially the same. The distal end insertion portion 106 and the treatment portion body 105 are made of insulating material, or they are produced by coating with insulating material.

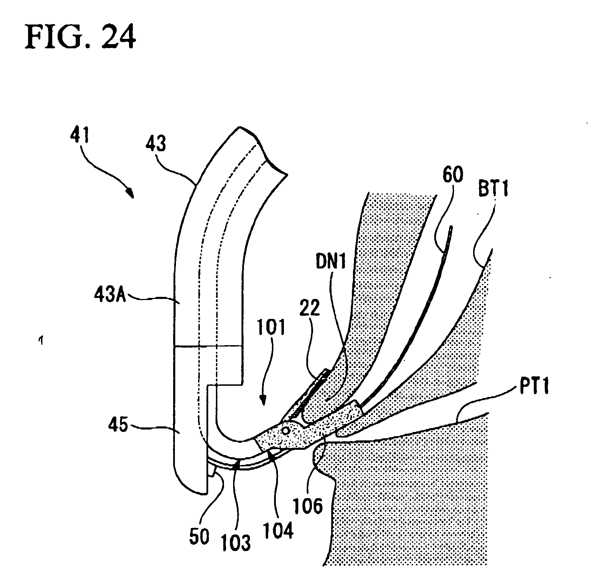

During medical treatment, the guide wire 60 is inserted in the duodenal papilla DN1 and the bile duct BT1 beforehand. The proximal end of the guide wire 60 is passed through the lumen 107 of the distal end insertion portion 106, and then the treatment instrument 101 is passed through the endoscope 41 and inserted in the body along the guide wire 60. As shown in FIG 24, the first forceps piece 22 opens inside the body, and the distal end insertion portion 106 is inserted in the duodenal papilla DN1 and the bile duct BT1 along the guide wire 60. After adjusting the insertion amount of the distal end insertion portion 106 and the direction of the rotation of the first forceps piece 22 around the axial line by advancement and retraction operation and rotational operation of the treatment instrument 101, the first forceps piece 22 is closed, and the duodenal papilla DN1 is held between the first forceps piece 22 and the distal end insertion portion 106. High frequency electric current is applied from a proximal side and the held portion of the duodenal papilla is cut out. The treatment instrument 101 is pulled out of the body while detaining the guide wire 60 and necessary medical treatments are performed by other treatment instruments.

According to the present embodiment, the number of lumens in the insertion portion 103 can be made one, the insertion portion becomes thinner and flexibility is improved. Insertion in the endoscope 41 or curving operation by the elevator 50 becomes easier, and inserting the distal end insertion portion 106 in the duodenal papilla DN1 becomes easier. The distal end insertion portion 106 is of substantially cylindrical shape and the shape is not incisive, therefore, even if bleeding occurs due to cut-out, the bleeding portion can be held by the first forceps piece 22 and the distal end insertion portion 106 without applying current, pressure can be applied on the bleeding portion, and bleeding can be stopped. Or, with the bleeding portion held as described above, a high frequency electric current for coagulation instead of a high frequency electric current for cut-out can be applied and the bleeding is stopped by coagulation without cut-out. The effects of cut-out are the same as in the first embodiment.

### (Fifth embodiment)

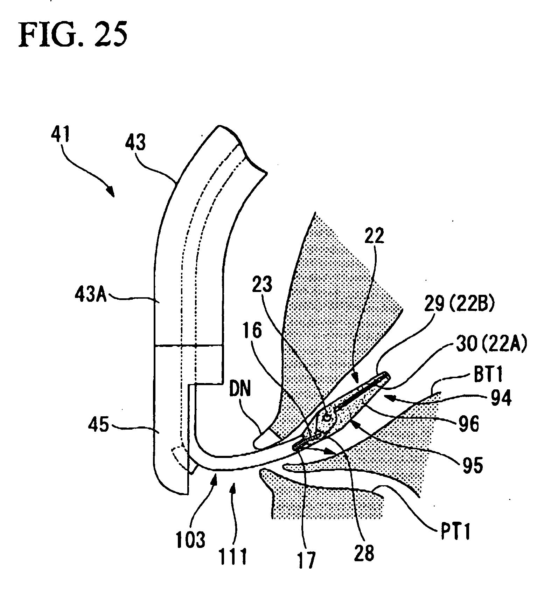

As shown in FIG 25, the treatment instrument for endoscope 111 (hereafter referred to as treatment instrument 111) is a monopolar cut-out forceps provided with a single-lumen type insertion portion 103. The distal end opening 17 of the lumen 14 is formed on the side of the distal end of the insertion portion 103. The lumen 14 has the operation wire 16 passed through therein so as to freely advance and retract and has such a diameter and opening area which enable the contrast agent to be injected in a state where the operation wire 16 is passed through.

The treatment portion 94 is fixed to the insertion portion 103, and a thinner type treatment portion body 95, which does not have a lumen therein, is used. Other than these configurations, similar configurations to the first embodiment are provided.

During medical treatment, the cut-out length is adjusted by the insertion amount of the distal end insertion portion 96 and the cut-out position is adjusted by rotating the treatment instrument 111, the duodenal papilla DN 1 is held by the treatment portion 94 and cut out by applying high frequency electric current. After the cut-out, the treatment portion 94 is inserted in the bile duct BT1. During X-ray photography, the contrast agent is injected in the bile duct BT1 or the like, as shown by the arrow in the figure, from the distal end opening 17 through the lumen 14.

According to the embodiment, the number of lumens is one, the insertion portion 103 can be made thinner, pulling out and insertion becomes easier, and curving to follow the shape of the endoscope 41 and the bile duct BT1 becomes easier as well. Moreover, as sthe distal end insertion portion 96 can be made thinner, it can also be easily inserted in the bile duct BT1. The distal end insertion portion 96 is of substantially cylindrical shape and the shape is not incisive, therefore, even if bleeding occurs due to cut-out, the bleeding portion can be held by the first forceps piece 22 and the distal end insertion portion 96 without applying current, pressure can be applied on the bleeding portion, and bleeding can be stopped. Or, with the bleeding portion held as described above, a high frequency electric current for coagulation instead of a high frequency electric current for cut-out can be applied and the bleeding can be stopped by coagulation without cut-out. The effects of cut-out are the same as in the first embodiment.

### (Sixth embodiment)

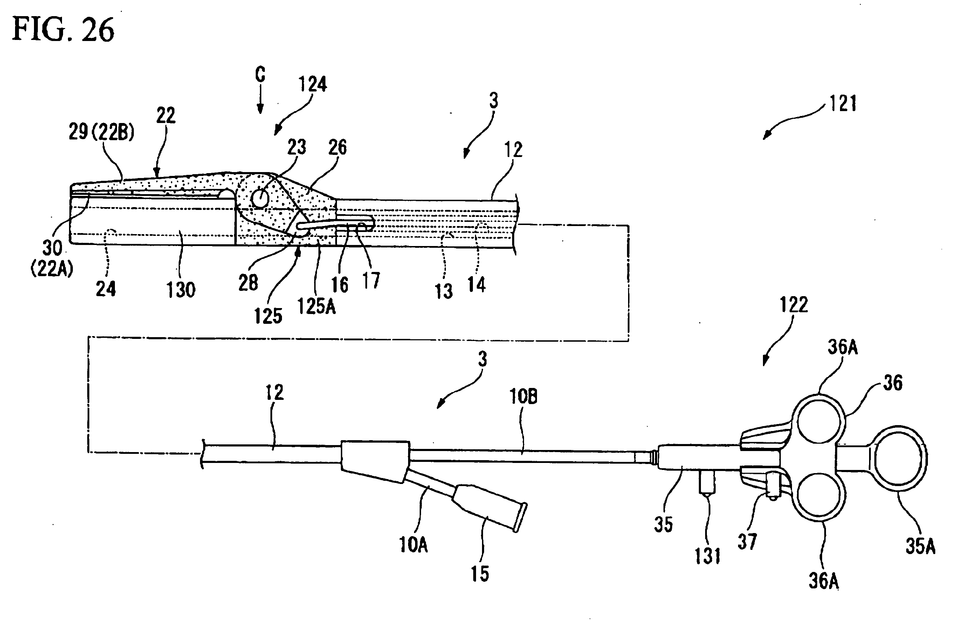

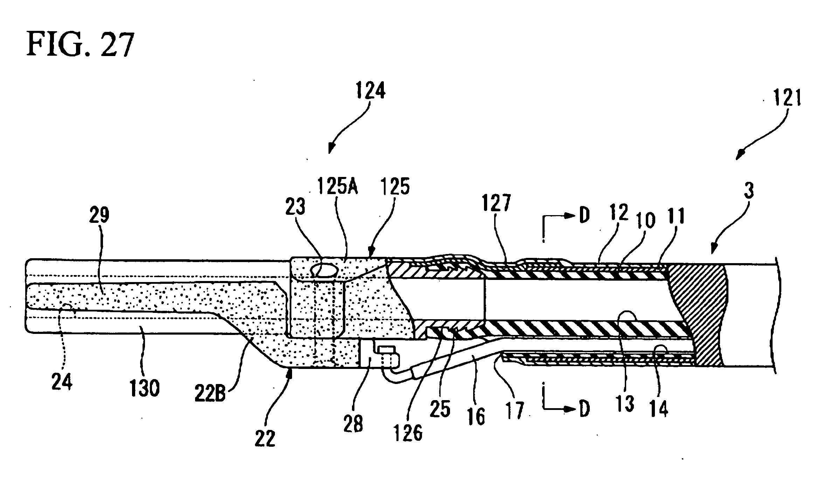

As shown in FIG 26 and FIG 27, treatment instrument for endoscope 121 (hereafter referred to as treatment instrument 121) has an operation portion 122 operated by the operator. A flexible insertion portion 3 extends from the operation portion 122, and a treatment portion 124 is provided at the distal end of the insertion portion 3. The treatment instrument 121 is a bipolar cut-out forceps provided with two electrodes in the treatment portion 124.

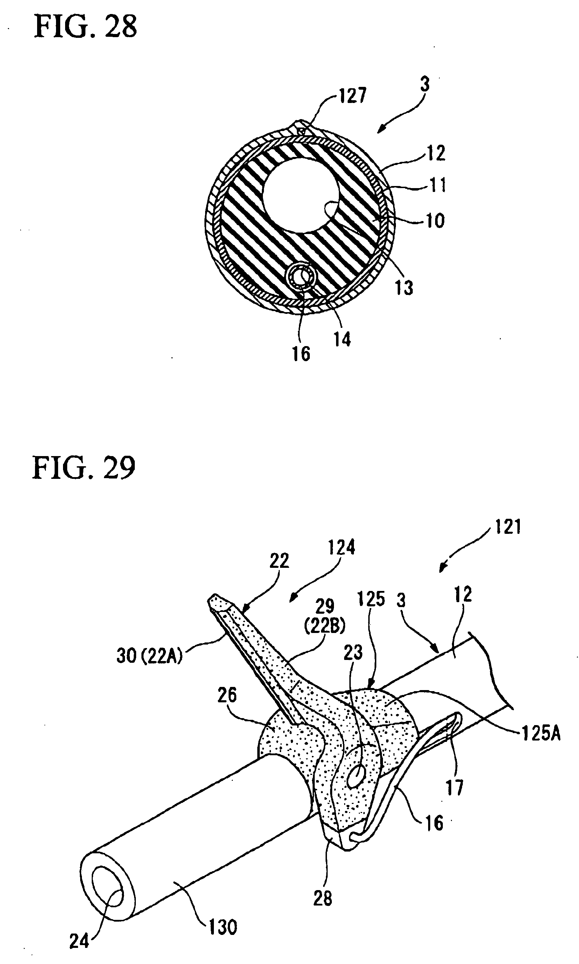

As shown in FIG 26 to FIG. 28, the insertion portion 3 has a flexible sheath 10 covered by braid 11 (second conductive member) made of metallic mesh, which is further covered by insulating tube 12. The first lumen 13 and the second lumen 14, the diameter of which is smaller than that of the first lumen 13, are formed in the sheath 10. The proximal end portion of the treatment portion 124 is inserted in the first lumen 13. An operation wire 16 is passed through the second lumen 14 so as be freely advanced or retracted. The operation wire 16 is configured with an insulating tube covering the first conductive member for permitting an application of high frequency electric current. The first conductive member is made of metallic wire with high straightness.

As shown in FIG 26, FIG 27, and FIG 29, the treatment portion 124 has a treatment portion body 125 of substantially cylindrical shape, which is made of a conductive material such as metal, fixed in the insertion portion 3. The treatment portion body 125 has a supporting portion 26 which protrudes in a protruding condition and the first forceps piece 22 is rotatably attached thereto by the pin 23. Furthermore, a distal end insertion portion 130 which is inserted first in the duodenal papilla is integrally extended at the distal end of the treatment portion body 125. The proximal end portion of the treatment portion body 125 is formed as a sheath linking portion 126 which is inserted in the sheath 10 of the insertion portion 3. On the outer periphery of the sheath linking portion 126, a return 25 to prevent the portion from being pulled out is provided in a protruding condition. The outer peripheral surface of the treatment portion body 125, except the distal end insertion portion 130 and the sheath linking portion 126, is covered by the insulating layer 125A. The sheath linking portion 126 and the braid 11 of the insertion portion 3 are electrically connected by the linking wire 127 (second conductive member).

The first forceps piece 22 has a linking portion 28 which links the operation wire at proximal end side than the pin 23, and distal end side than the pin 23 extends in elongated manner along the axial line. The distal end portion has a triangular shape cross sectional view, and the vertex portion directed toward the distal end insertion portion 130 forms the cut-out electrode 30. The first forceps piece 22, except the cut-out electrode 30 and the linking portion 28, is covered by the insulating layer 22B. The pin 23 is either made of an insulating material, or covered by an insulating material.

The distal end insertion portion 130 extends in the longitudinal direction of the insertion portion 3, that is, along the insertion direction, and it is a portion which receives the first forceps piece 22 forming a second forceps piece which holds the biological tissue. The distal end insertion portion 130 is of a substantially cylindrical shape in which a lumen 24 is formed which links to the first lumen 13 of the insertion portion 3. A distal end position of the distal end insertion portion 130 and a distal end position of the first forceps piece 22 are substantially the same.

The conductive material, insulating material, and covering material used in the treatment portion 124 may be the same as those used in the first embodiment.

As shown in FIG 26, the operation portion 122 includes a slider 36 which is attached to the operation portion body 35 so as to freely advance and retract. The operation wire 16 is fixed to the slider 36, The first conductive member of the operation wire 16 is connected to the first electric connector 37. The second electric connector 131 is provided in the operation portion body 35. The braid 11, which is the second conductive member, is connected to the second electric connector 131. These electric connectors 37, 131 are each connected to the each of the two bipolar output terminals of the high frequency power supply (not shown in the figures).

Next, the actions of the present embodiment are described hereafter.

The treatment instrument 121 is made to protrude toward the duodenal papilla DN1 from the endoscope 41. With the first forceps piece 22 in the open condition, the entire treatment instrument 121 is advanced and the distal end insertion portion 130 is inserted first in the duodenal papilla DN1. The insertion amount of the distal end insertion portion 130 is adjusted by the insertion amount of the treatment instrument 121 relative to the endoscope 41. Furthermore, by rotating the entire treatment instrument 121, the position of the first forceps piece 22, that is the direction of cut-out, is adjusted.

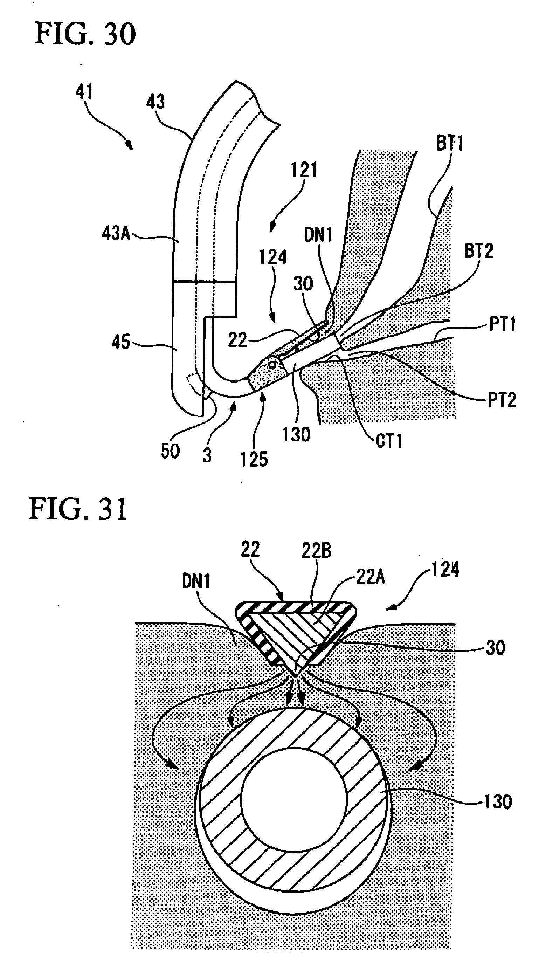

The first forceps piece 22 is closed by operating the operation portion 122. As shown in FIG 30, after holding the duodenal papilla DN1 by the first forceps piece 22 and the distal end insertion portion 130, high frequency electric current is output from the high frequency power supply. As shown in FIG 31, high frequency electric current flows between the cut-out electrode 30 of the first forceps piece 22 and the distal end insertion portion 130 through the tissue. As the electric current density around the elongated cut-out electrode 30 increases, the biological tissue which is in contact with the cut-out electrode 30 is cut out. On the other hand, as the entire outer peripheral surface of the distal end insertion portion 130 is a conductive portion, the electric current density does not increase. Therefore, the biological tissue all over the surface of the distal end insertion portion 130 will not be cut out.

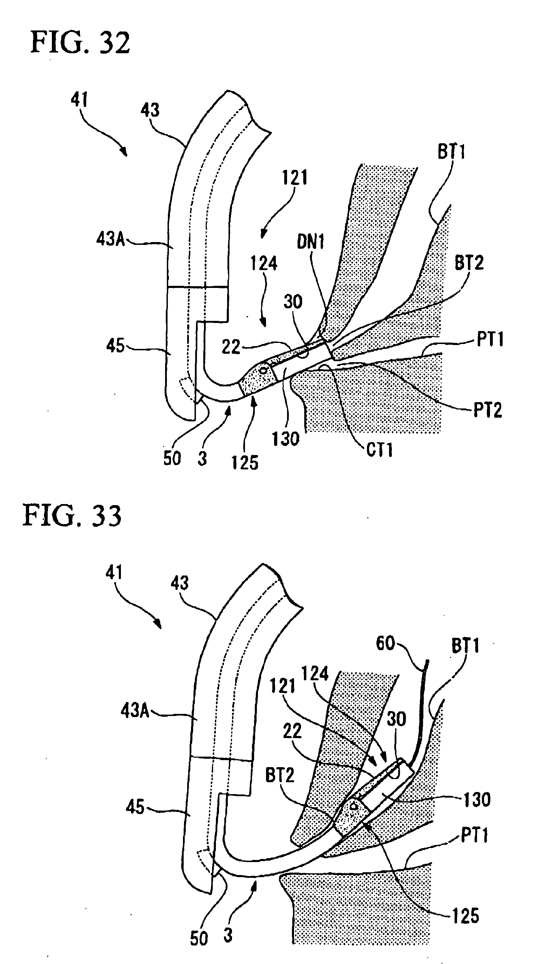

As shown in FIG 32, when the duodenal papilla DN1 is cut out, the first forceps piece 22 is closed by operating the operation portion 122, and the treatment portion 124 is inserted in the bile duct BT1. If necessary, the guide wire 60 is inserted, as shown in FIG. 33. The contrast agent is injected for imaging. When replacing the treatment instrument, the guide wire 60 is remained as it is, the treatment instrument 121 is pulled out, and then other treatment instruments are inserted in the bile duct BT1 using the guide wire 60 as the guide. When the required medical treatment is completed, other treatment instruments, the guide wire 60, and the endoscope 41 are each pulled out of the body.

Before inserting the treatment instrument 121, the guide wire 60 may be inserted in the bile duct BT1 from the duodenal papilla DN1. The treatment instrument 121 may be inserted so as to pass the guide wire 60 through the first lumen 13, and the distal end insertion portion 130 may be inserted in the duodenal papilla DN1 along the guide wire 60.

According to the present embodiment, a procedure of attaching the return electrode in the patient as required in the monopolar type, can be avoided. The distal end insertion portion 130 is of substantially cylindrical shape and the shape is not incisive, therefore, even if bleeding occurs due to cut-out, the bleeding portion can be held by the first forceps piece 22 and the distal end insertion portion 130 without applying current, pressure can be applied on the bleeding portion, and bleeding can be stopped. Or, with the bleeding portion held as described above, a high frequency electric current for coagulation instead of a high frequency electric current for cut-out can be applied and the bleeding is stopped by coagulation without cut-out Thus, the effects of easy and accurate cut-out of the duodenal papilla are similar to those of the first embodiment

### (Seventh embodiment)

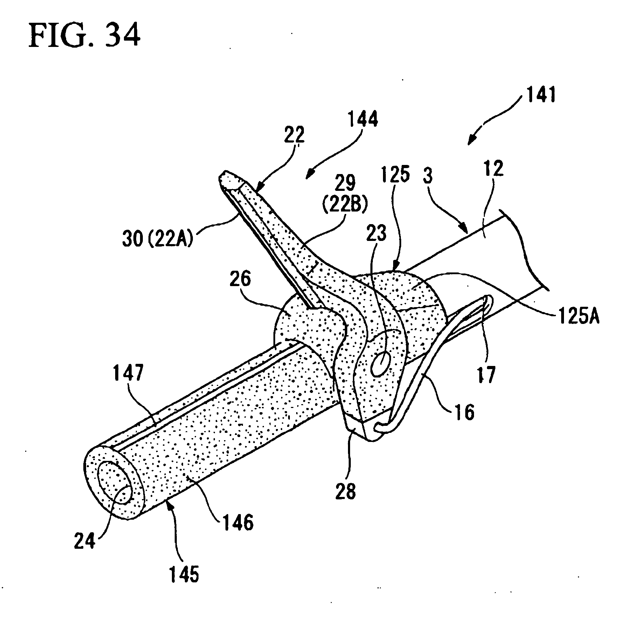

As shown in FIG 34, the treatment instrument for endoscope 141 (hereafter referred to as treatment instrument 141) is a bipolar cut-out forceps in which a distal end insertion portion 145 of a treatment portion 144 is configured such that a conductive member of cylindrical shape is covered by insulating layer 146 except for the position facing the cut-out electrode 30 of the first forceps piece 22. The rest of the configuration is the same as that of the sixth embodiment.

An exposed electrode 147 formed on the distal end insertion portion 145 as a non-insulated portion has approximately the same length and width as the cut-out electrode 30. The exposed electrode 147 is connected to the second electric connector 131 (see FIG 26) of the operation portion 122 through the distal end insertion portion 145 and the treatment portion body 125, the linking wire 127 (see FIG. 27), and the braid 11 (see FIG 27). The distal end position of the distal end insertion portion 145 coincides substantially with the distal end position of the first forceps piece 22. The lumen 24 in the distal end insertion portion 145 is also covered by the insulating layer 146. The material of the insulating layer 146 is the same as that in the first embodiment.

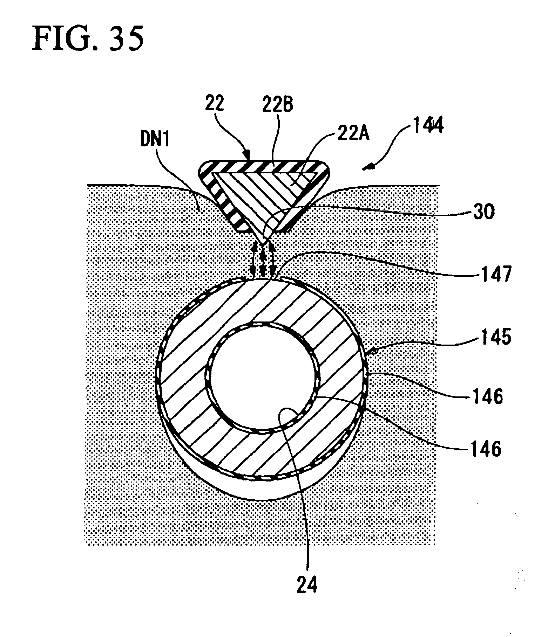

With this treatment instrument 141, medical treatment is given by the same procedure as in the fifth embodiment. When pre-cutting the duodenal papilla DN1, the biological tissue is held by the distal end insertion portion 145 inserted in the duodenal papilla DN1 and the first forceps piece 22. High frequency electric current is applied to the first forceps piece 22 and the distal end insertion portion 145. As shown in FIG. 35, the electric current density increases in the elongated cut-out electrode 30 and in the exposed electrode 147 respectively, and the biological tissue between the cut-out electrode 30 and the exposed electrode 147 is cut out.

According to the present embodiment, as an intensive high frequency electric current flows in the vicinity of the elongated cut-out electrode 30 and the exposed electrode 147, the biological tissue which is in contact with other portions of the treatment portion 144 receives practically no effect of the high frequency electric current. By aligning the shape of the exposed electrode 147 with the shape of the cut-out electrode 30, the cut-out position and the cut-out amount can be further controlled. However, the shape of the exposed electrode 147 is not necessarily coincided with the shape of the cut-out electrode 30. The distal end insertion portion 145 is of substantially cylindrical shape and the shape is not incisive, therefore, even if bleeding occurs due to cut-out, the bleeding portion can be held by the first forceps piece 22 and the distal end insertion portion 145 without applying current, pressure can be applied on the bleeding portion, and bleeding can be stopped. Or, with the bleeding portion held as described above, a high frequency electric current for coagulation instead of a high frequency electric current for cut-out can be applied and the bleeding is stopped by coagulation without cut-out. Other effects are the same as those in the fifth embodiment.

### (Eighth embodiment)

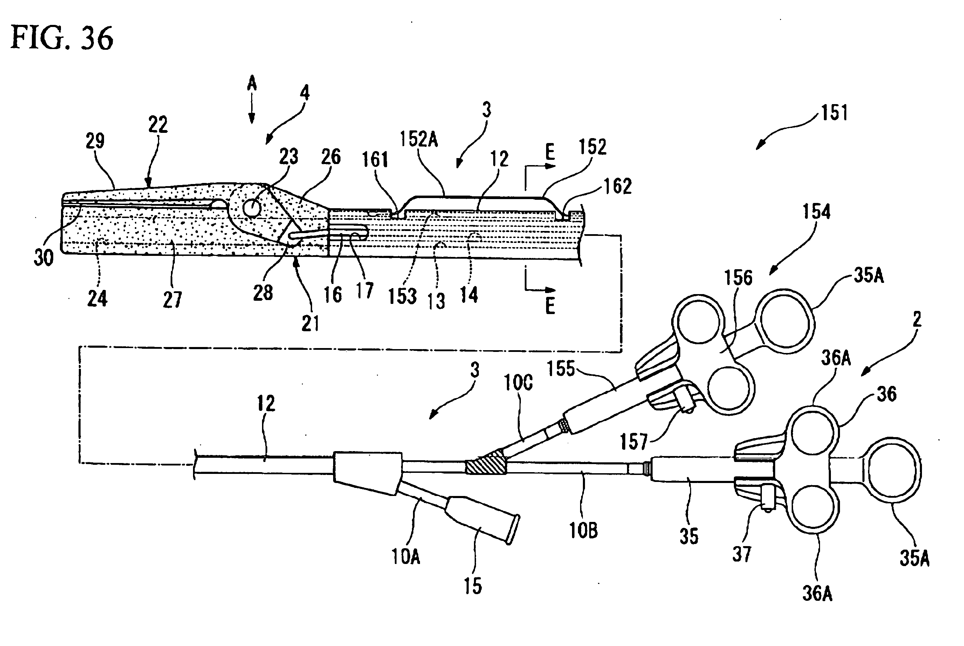

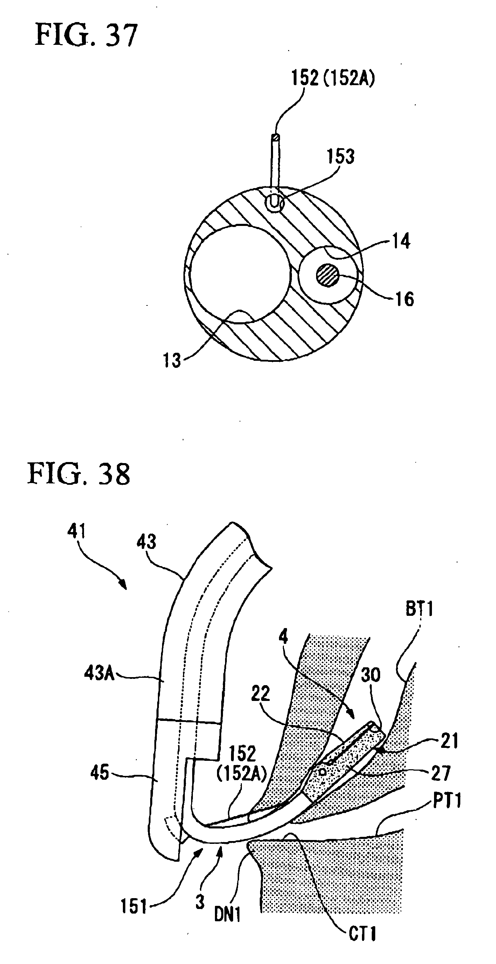

As shown in FIG. 36 and FIG 37, the treatment instrument for endoscope 151 (hereafter referred to as treatment instrument 151) is a monopolar cut-out forceps having the configuration below, in addition to the configuration of the first embodiment

The insertion portion 3 has three lumens 13, 14, 153, and the cut-out wire 152 passes through the third lumen 153. A first side opening 161 opening to the outside and a second side opening 162 opening to the outside at proximal end side than the first side opening 161 are provided at the distal end portion of the third lumen 153. The distal end portion of the cut-out wire 152 comes out from the second side opening 162 first, then returns into the third lumen 153 from the first side opening 161, and is fixed in the third lumen 153.

The treatment instrument 151 has a second operation portion 154 used as an operation portion for operating the cut-out wire 152. The second operation portion 154 has an operation portion body 155 fixed to the tube 10C which is linked to the third lumen 153. A second slider 156 is attached to the operation portion body 155 so as to freely advance and retract. A proximal side of the cut-out wire 152 is inserted in the second slider 156. The cut-out wire 152 is linked to the second electric connector 157 provided in the second slider 156. The second electric connector 157 is independent of the electric connector 37 (first electric connector) on the first operation portion 2 side and it can be connected to the high frequency power supply which is not shown in the figures.

Similar to the first embodiment, the treatment instrument 151 cuts out the duodenal papilla DN1 and the entrance BT2 of the bile duct BT1, but in case the cut-out of the entrance BT2 of the bile duct BT1 is insufficient, to dissect up to the back of the inside cavity of the bile duct BT1, the second slider 156 is pulled, and as shown in FIG 38, the exposed portion 152A outside the cut-out wire 152 is stretched out like the string in a bow and the inside cavity of the bile duct BT1 is additionally cut out. When the second slider 156 is not operated, the cut-out wire 152 will not be in the stretched condition, therefore, there is no effect when the treatment instrument 151 is inserted in the endoscope 41 or in the duodenal papilla DN1 or pulled out from them.

According to the present embodiment, even if the cut-out of the back of the inside cavity of the bile duct BT1 is inadequate, additional cut-out can be performed without replacing to a conventional papillotome, accordingly the time required for procedure can be shortened and the cost can be cut down. The distal end insertion portion 27 is of substantially cylindrical shape and the shape is not incisive, therefore, even if bleeding occurs due to cut-out, the bleeding portion can be held by the first forceps piece 22 and the distal end insertion portion 27 without applying current, pressure can be applied on the bleeding portion, and bleeding can be stopped. Or, with the bleeding portion held as described above, a high frequency electric current for coagulation instead of a high frequency electric current for cut-out can be applied and the bleeding is stopped by coagulation without cut-out. Other effects are the same as those in the first embodiment.

It is to be noted that the present invention is not limited to the embodiments mentioned above and can be widely used.

For instance, the first conductive member may be provided separately from the operation wire 16. The second conductive member is not limited to the braid which covers all circumference of the sheath 10 but wire rod or the like may also be used.

Curved portion 82 and bent portion 87 may be provided in the treatment instruments other than the treatment instrument of the second embodiment. The shapes of the treatment instruments 121, 141 of the bipolar type may also be used in the configuration of the third to fifth embodiments, or in the configuration of the eighth embodiment.

## Claims

1. A treatment instrument for an endoscope (1, 81, 91, 101, 111, 121, 141, 151) including:
an insertion portion (3, 103), which is elongated and flexible, having a lumen (13, 14, 71, 153) formed therein, and being provided with a torque transmission ability for transmitting a rotation at a proximal side thereof to a distal side thereof;
a distal end portion provided in a distal end of the insertion portion (3, 103) comprising:
a supporting portion (26) fixed relative to the insertion portion (3, 103);
a first forceps piece (22) being pivotably supported by a pin (23) in the supporting portion (26), and provided with a cut-out electrode (30);
a first conductive member electrically connected to the cut-out electrode (30) of the first forceps piece (22) and connected to a power supply device by passing through the insertion portion (3, 103); and
a distal end insertion portion (27, 96, 106, 130, 145) which becomes a second forceps piece that receives the first forceps piece provided distal to the supporting portion (26), **characterized in that**
the distal end insertion portion (27, 96, 106, 130, 145) is communicated with the lumen (13, 14, 71, 153) of the insertion portion (3 ,103) and is of a substantially cylindrical shape in which a lumen (24, 107) is formed;
a distal end of the lumen (24, 107) opens at a distal end of the distal end insertion portion (27, 96, 106,130,145); and
a distal end position of the distal end insertion portion (27, 96, 106, 130, 145) substantially coincides with the distal end position of the first forceps piece (22).

2. The treatment instrument for an endoscope according to Claim 1, wherein
the first forceps piece has a cut-out electrode which protrudes while reducing its width toward the distal end insertion portion;
the cut-out electrode is covered by insulation except for the distal end portion facing the distal end insertion portion; and
the distal end insertion portion is insulated from a tissue and the cut-out electrode of the first forceps piece which is protruding.

3. The treatment instrument for an endoscope according to Claim 1, wherein the distal end insertion portion is made of a conductive material, and is configured such that it can be connected to a power supply device through a second conductive member disposed in the insertion portion.

4. The treatment instrument for an endoscope according to one of Claims 1 to 3, wherein
an electrode is formed by insulating the distal end insertion portion except a part facing the first forceps piece.

5. The treatment instrument for an endoscope according to Claim 4, wherein the electrode portion which is elongatedly formed in the distal end insertion portion along the longitudinal direction thereof, to suit the cut-out electrode of the first forceps piece.

6. The treatment instrument for an endoscope according to one of the preceding Claims, wherein
a posterior end of the lumen (24, 107) of the distal end insertion portion opens proximal to a posterior end of the cut-out electrode (30).

7. The treatment instrument for an endoscope (1, 81, 91, 101, 111, 121, 141, 151) according to Claim 6, wherein
the insertion portion (3, 103) has two or more lumens (13, 14, 71, 153);
the second lumen (14) extends to a proximal operation portion (2) and is inserted by a first conductive member; and
the first lumen (13) is communicated with the lumen (24, 107) of the distal end insertion portion (27, 96, 106, 130, 145), wherein
a posterior end of the lumen (24, 107) opens proximal to a connection position between the supporting portion (26) and the insertion portion (3, 103).

8. The treatment instrument for an endoscope according to Claim 7, wherein
the insertion portion (3, 103) has a third lumen (71, 153), and wherein
the third lumen (71, 153) opens proximal to the insertion portion (3, 103) and is communicated with the first lumen (13) at the distal end side of the insertion portion (3, 103).

## Patentansprüche

1. Behandlungsinstrument für ein Endoskop (1, 81, 91, 101, 111, 121, 141, 151), mit:
einem Einführabschnitt (3, 103), der länglich und flexibel ist und ein darin ausgebildetes Lumen (13, 14, 71, 153) hat, und der über die Fähigkeit der Drehmomentübertragung verfügt, um eine Rotation an seiner proximalen Seite zu seiner distalen Seite zu übertragen;
einem distalen Endabschnitt, der im distalen Ende des Einführabschnitts (3, 103) angeordnet ist, aufweisend:
einen Halterungsabschnitt (26), der relativ zum Einführabschnitt (3, 103) fest angeordnet ist;
ein erstes Zangenteil (22), das um einen Stift (23) im Halterungsabschnitt (26) schwenkbar gelagert und mit einer Ausschneid-Elektrode (30) versehen ist;
ein erstes leitfähiges Element, das elektrisch mit der Ausschneid-Elektrode (30) des ersten Zangenteils (22) und mit einem Spannungsversorgungsgerät verbunden ist, indem es durch den Einführabschnitt (3, 103) geführt wird; und
einen Einführabschnitt (27, 96, 106, 130, 145) am distalen Ende, der ein zweites Zangenteil wird, das das erste Zangenteil aufnimmt, das distal am Halterungsabschnitt (26) vorgesehen ist,
**dadurch gekennzeichnet, dass**
der Einführabschnitt (27, 96, 106, 130, 145) am distalen Ende mit dem Lumen (13, 14, 71, 153) des Einführabschnitts (3, 103) kommuniziert und eine im Wesentlichen zylindrische Form hat, in der ein Lumen (24, 107) ausgebildet ist;
das distale Ende des Lumens (24, 107) in das distale Ende des Einführabschnitts (27, 96, 106, 130, 145) am distalen Ende mündet; und
eine distale Endposition des Einführabschnitts (27, 96, 106, 130, 145) am distalen Ende im Wesentlichen mit der distalen Endposition des ersten Zangenteils zusammenfällt.

2. Behandlungsinstrument für ein Endoskop nach Anspruch 1, bei dem
das erste Zangenteil eine Ausschneid-Elektrode hat, die herausragt, während ihre Breite zum Einführabschnitt am distalen Ende abnimmt;
die Ausschneid-Elektrode mit Ausnahme des zum Einführabschnitt am distalen Ende weisenden distalen Endabschnitts mit einer Isolierung bedeckt ist; und
der Einführabschnitt am distalen Ende gegenüber Gewebe und der herausragenden Ausschneid-Elektrode des ersten Zangenteils isoliert ist.

3. Behandlungsinstrument für ein Endoskop nach Anspruch 1, bei dem der Einführabschnitt am distalen Ende aus einem leitfähigen Material besteht und so konfiguriert ist, dass er über ein im Einführabschnitt angeordnete zweites leitfähiges Element mit einem Spannungsversorgungsgerät verbunden werden kann.

4. Behandlungsinstrument für ein Endoskop nach einem der Ansprüche 1 bis 3, bei dem eine Elektrode durch Isolieren des Einführabschnitts mit Ausnahme eines zum ersten Zangenteil weisenden Teils durch Isolieren gebildet ist.

5. Behandlungsinstrument für ein Endoskop nach Anspruch 4, bei dem der im Einführabschnitt am distalen Ende in dessen Längsrichtung längliche Elektrodenabschnitt so geformt ist, dass er mit der Ausschneid-Elektrode des ersten Zangenteils übereinstimmt.

6. Behandlungsinstrument für ein Endoskop nach einem der vorigen Ansprüche, bei dem
ein posteriores Ende des Lumens (24, 107) des Einführabschnitts am distalen Ende proximal in ein posteriores Ende der Ausschneid-Elektrode (30) mündet.

7. Behandlungsinstrument für ein Endoskop (1, 81, 91, 101, 111, 121, 141, 151) nach Anspruch 6, bei dem
der Einführabschnitt (3, 103) zwei oder mehr Lumen (13, 14, 71, 153) hat;
sich das zweite Lumen (14) zu einem proximalen Betätigungsabschnitt (2) erstreckt und durch ein erstes leitfähiges Element eingeführt wird; und
bei dem das erste Lumen (13) mit dem Lumen (24, 107) des Einführabschnitts (27, 96, 106, 130, 145) am distalen Ende kommuniziert, wobei
ein posteriores Ende des Lumens (24, 107) proximal in eine Verbindungsposition zwischen dem Halterungsabschnitt (26) und dem Einführabschnitt (3, 103) mündet.

8. Behandlungsinstrument für ein Endoskop nach Anspruch 7, bei dem der Einführabschnitt (3, 103) ein drittes Lumen (71, 153) hat, und bei dem das dritte Lumen (71, 153) proximal in den Einführabschnitt (3, 103) mündet und mit dem ersten Lumen (13) am Ende der distalen Seite des Einführabschnitt (3, 103) kommuniziert.

## Revendications

1. Instrument de traitement pour un endoscope (1, 81, 91, 101, 111, 121, 141, 151) comprenant :
une partie d'insertion (3, 103) qui est allongée et flexible, comportant une lumière (13, 14, 71, 153) formée dans celle-ci, et possédant une capacité de transmission de couple destinée à transmettre une rotation au niveau d'un côté proximal de celle-ci vers un côté distal de celle-ci ;
une partie d'extrémité distale prévue dans une extrémité distale de la partie d'insertion (3, 103) comprenant :
une partie de support (26) fixée par rapport à la partie d'insertion (3, 103) ;
une première partie de pince (22) étant supportée de manière pivotante par un axe (23) dans la partie de support (26), et munie d'une électrode de découpe (30) ;
un premier élément conducteur connecté électriquement à l'électrode de découpe (30) de la première partie de pince (22) et connecté à un dispositif d'alimentation en passant à travers la partie d'insertion (3, 103) ; et
une partie d'insertion d'extrémité distale (27, 96, 106, 130, 145) qui devient une deuxième partie de pince qui reçoit la première partie de pince prévue de façon distale par rapport à la partie de support (26), **caractérisé en ce que**
la partie d'insertion d'extrémité distale (27, 96, 106, 130, 145) est en communication avec la lumière (13, 14, 71, 153) de la partie d'insertion (3, 103) et est d'une forme sensiblement cylindrique dans laquelle une lumière (24, 107) est formée ;
une extrémité distale de la lumière (24, 107) s'ouvre au niveau d'une extrémité distale de la partie d'insertion d'extrémité distale (27, 96, 106, 130, 145) ; et
une position de l'extrémité distale de la partie d'insertion d'extrémité distale (27, 96, 106, 130, 145) coïncide sensiblement avec la position de l'extrémité distale de la première partie de pince (22).

2. Instrument de traitement pour un endoscope selon la revendication 1, dans lequel la première partie de pince comporte une électrode de découpe qui fait saillie tout en réduisant sa largeur vers la partie d'insertion d'extrémité distale ;
l'électrode de découpe est recouverte par un isolant excepté pour la partie d'extrémité distale en regard de la partie d'insertion d'extrémité distale ; et
la partie d'insertion d'extrémité distale est isolée d'un tissu et de l'électrode de découpe de la première partie de pince qui fait saillie.

3. Instrument de traitement pour un endoscope selon la revendication 1, dans lequel la partie d'insertion d'extrémité distale est composée d'un matériau conducteur, et est configurée d'une manière telle qu'elle peut être connectée à un dispositif d'alimentation par l'intermédiaire d'un deuxième élément conducteur disposé dans la partie d'insertion.

4. Instrument de traitement pour un endoscope selon l'une quelconque des revendications 1 à 3, dans lequel
une électrode est formée en isolant la partie d'insertion d'extrémité distale excepté une partie en regard de la première partie de pince.

5. Instrument de traitement pour un endoscope selon la revendication 4, dans lequel la partie d'électrode est formée de manière allongée dans la partie d'insertion d'extrémité distale le long de la direction longitudinale de celle-ci pour s'adapter à l'électrode de découpe de la première partie de pince.

6. Instrument de traitement pour un endoscope selon l'une quelconque des revendications précédentes, dans lequel
une extrémité postérieure de la lumière (24, 107) de la partie d'insertion d'extrémité distale s'ouvre de façon proximale par rapport à une extrémité postérieure de l'électrode de découpe (30).

7. Instrument de traitement pour un endoscope (1, 81, 91, 101, 111, 121, 141, 151) selon la revendication 6, dans lequel
la partie d'insertion (3, 103) comporte deux lumières (13, 14, 71, 153) ou plus;
la deuxième lumière (14) s'étend vers une partie d'actionnement proximale (2) et est insérée par un premier élément conducteur ; et
la première lumière (13) est en communication avec la lumière (24, 107) de la partie d'insertion d'extrémité distale (27, 96, 106, 130, 145), dans lequel
une extrémité postérieure de la lumière (24, 107) s'ouvre de façon proximale par rapport à une position de connexion entre la partie de support (26) et la partie d'insertion (3, 103).

8. Instrument de traitement pour un endoscope selon la revendication 7, dans lequel
la partie d'insertion (3, 103) comporte une troisième lumière (71, 153), et dans lequel
la troisième lumière (71, 153) s'ouvre de façon proximale par rapport à la partie d'insertion (3, 103) et est en communication avec la première lumière (13) au niveau du côté d'extrémité distale de la partie d'insertion (3, 103).
